# EUROPÄISCHE PATENTSCHRIFT

(11) **EP 1 013 297 B1**
(45) Veröffentlichungstag und Bekanntmachung des Hinweises auf die Patenterteilung: **30.10.2002**
(21) Anmeldenummer: 99125543.1
(22) Anmeldetag: 21.12.1999
(51) Int. Cl.: A61M 5/30

(54) **Ejektionsgerät zur Hochdruckejektion einer Flüssigkeit**
High pressure jet injector for liquids
Injecteur à haute pression pour liquides

(30) Priorität: 21.12.1998 DE 19859137
(43) Veröffentlichungstag der Anmeldung: 28.06.2000
(73) Patentinhaber: Ferton Holding SA, 2800 Delémont (CH)
(72) Erfinder: Menne, Andreas Dr., 88709 Meersburg (DE); Merkle, Wolfgang, 88709 Meersburg (DE)
(74) Vertreter: Viering, Jentschura & Partner

(56) Entgegenhaltungen:
- DE-A- 2 035 098
- US-A- 2 605 763
- US-A- 3 189 029
- US-A- 5 505 697

## Beschreibung

Die Erfindung betrifft ein Ejektionsgerät zur Hochdruckejektion einer Flüssigkeit, oder einer Feststoffpartikel enthaltenden Flüssigkeit, mit einer die Flüssigkeit aufnehmenden Druckkammer, die in eine Ejektionsöffnung ausmündet und die von einem Arbeitskolben begrenzt wird. Von dem Arbeitskolben ist, durch Übertragung eines vorzugsweise elastischen Stoßes auf das druckkammerferne Ende des Arbeitskolbens, eine Kompressionswelle übertragbar, durch welche das druckkammerseitige Ende des Arbeitskolbens in die Druckkammer um einen vorbestimmten Verlagerungshub unter Reduzierung von deren Volumen ausgelenkt wird. Hierbei ist die Volumenreduktion der Druckkammer wesentlich kleiner als das Druckkammervolumen. Das Ejektionsgerät hat ferner ein Antriebsstück, von dem der elastische Stoß erzeugbar ist.

Ein derartiges Ejektionsgerät ist beispielsweise aus der EP 0 771 219 bekannt. Es ist, wie aus der EP 0 771 219 ersichtlich, insbesondere zur Ejektion genau dosierter, kleinster Flüssigkeitsmengen, ggf. Kubikmillimeter, vorgesehen und eignet sich demnach insbesondere als medizinisches Gerät zur Injektion sehr kleiner und genau einzuhaltender Medikamenten-Flüssigkeitsdosen.

Das Ejizieren derart kleiner Flüssigkeitsmengen wird auch gemäß der Erfindung dadurch erzielt, daß von dem Antriebsstück lediglich ein elastischer Stoß auf den Arbeitskolben übertragen wird und dieser dann nicht weiter von dem Antriebsstück in die Druckkammer bewegt wird. Das heißt, nach erfolgter Stoßübertragung wird von dem Antriebsstück auf den. Arbeitskolben keine äußere Antriebskraft mehr ausgeübt, so daß die von dem Arbeitskolben begrenzte Flüssigkeit in der Druckkammer nicht wie bei einer gewöhnlichen Spritze ausgedrückt wird. Stattdessen wird das Ejizieren der Flüssigkeit lediglich von der durch die Stoßübertragung im Arbeitskolben angeregten Kompressionswelle erreicht, wodurch der Arbeitskolben einen Hochdruckimpuls auf die in der Druckkammer vorliegende Flüssigkeit überträgt und eine Hochdruckejektion aus der Ejektionsöffnung bewirkt. Der Ejektionsdruck und damit das ejizierte Flüssigkeitsvolumen sind durch Einstellung der Antriebsgeschwindigkeit des Antriebsstücks genau einstellbar, da die Größe des übertragenen Impulses von dieser Antriebsgeschwindigkeit abhängig ist.

Auch gemäß dem in der EP 0 771 219 beschriebenen Ejektionsgerät ist das Druckkammervolumen wesentlich größer als das Hubvolumen des Arbeitskolbens, um unabhängig von der Größe der Druckkammer eine kleine, genau dosierte Menge der Flüssigkeit ejizieren zu können.

Um zu verhindern, daß das Antriebsstück nach der Stoßimpulsübertragung noch eine äußere Kraft auf den Arbeitskolben ausübt, kann das Antriebsstück beispielsweise nur bis zum Beginn der Stoßübertragung in Richtung zu dem Arbeitskolben angetrieben werden oder durch einen Anschlag an einer nach dem Stoß erfolgenden Kraftübertragung auf den Arbeitskolben gehindert sein.

Der Arbeitskolben ist vorzugsweise aus einem festen Material, von dem elastische Stoßwellen möglichst verlustfrei übertragen werden, wie z.B. einem Metallmaterial. Das Antriebsstück kann irgendein stoßübertragendes Bauteil sein, z.B. ein Antriebskolben, der in einem Antriebsrohr angetrieben beschleunigbar ist und bevorzugt zu dem Arbeitskolben koaxial ausgerichtet ist. Das Antriebsstück kann aber auch anders als ein Kolben gestaltet sein, beispielsweise als Platte oder Kipphebel, die/der auf den Arbeitskolben aufschlägt und dadurch den Antriebsstoß ausübt.

Das Antriebsstück kann z.B. pneumatisch, hydraulisch, mechanisch oder elektromagnetisch angetrieben sein. Hierbei kann der Antrieb des Antriebsstücks derart gestaltet sein, daß dieses für lediglich einen Antriebshub antreibbar ist. Bevorzugt ist das Antriebsstück jedoch zur aufeinanderfolgenden Übertragung von Stoßimpulsen in periodischer Wiederholung antreibbar. Dadurch kann die Gesamtejektionsmenge in Abhängigkeit von der Anzahl der Wiederholungen gesteuert werden. Bevorzugt ist das Antriebsstück als ein entlang einer Beschleunigungsstrecke antreibbares Bauteil vorgesehen.

Bei dem aus der EP 0 771 219 bekannten Ejektionsgerät wird bei dessen periodischem Betrieb nach erfolgter Flüssigkeitsejektion der Arbeitskolben aufgrund den Reflektionen der elastischen Stoßwelle am freien Kolbenende und der Druckwelle am Ejektionsende der Druckkammer wieder zurück in seine vor dem elastischen Stoß vorliegende Ausgangsposition zurückgeführt. Durch die dadurch bewirkte Druckabsenkung in der Druckkammer wird Flüssigkeit aus einem Vorratsbehälter in die Druckkammer nachgesaugt, so daß das Gerät für die nächste Ejektion sofort wieder bereit ist und die Flüssigkeit in vorbestimmten Dosen aufeinanderfolgend ejiziert werden kann.

Durch die Erfindung wird ein Ejektionsgerät der eingangs erwähnten Art geschaffen, das von den vorstehend beschriebenen Grundprinzipien Gebrauch macht, jedoch unabhängig von einer Flüssigkeitszufuhr periodisch betreibbar ist.

Das erfindungsgemäße Ejektionsgerät ist derart ausgebildet, daß der Arbeitskolben nach der Verlagerung seines vorlaufenden Endes nicht wieder in seine Ausgangslage vor der Übertragung des wenigstens teilweise elastischen Stoßes zurückverlagert wird, sondern durch aufeinanderfolgend auf ihn ausgeübte, elastische Stöße sukzessive weiter jeweils um den vorbestimmten Verlagerungshub in die Druckkammer hinein verlagert wird.

Wie bei den in den Druckschriften DE 2035098 A und US-A-2 605 763 offenbarten Vorrichtungen wird bei dem erfindungsgemäßen Ejektionsgrät der Arbeitskolben bei jeder Stoßübertragung unter Ejizieren von Flüssigkeit ein Stück, d.h. um den vorbestimmten Arbeitshub, in die Druckkammer hinein verlagert. Als Flüssigkeitsvorrat dient die anfänglich in der Druckkammer vorliegende Flüssigkeit, welche nach und nach aus der Druckkammer ausgespritzt wird. Bei dem erfindungsgemäßen Ejektionsgerät wird die Druckkammer somit gleichzeitig als Flüssigkeitsvorratskammer verwendet, so daß ein zusätzliches Vorratsbehältnis inklusive Zuführleitungen, welche das Vorratsbehältnis mit der Druckkammer verbinden, und ggf. Rückschlagventile, von denen ein Zurückfließen von Flüssigkeit aus der Druckkammer in das Vorratsbehältnis verhindert wird, entfallen können.

Um zu verhindern, daß der Arbeitskolben durch eine teilweise reflektierte Kompressionswelle und die Druckerhöhung in der Druckkammer wieder ein Stück aus Druckkammer heraus zurückwandert, was ein Nachsaugen von Luft in die Druckkammer und einen ungenaueren Betrieb des Ejektionsgeräts zur Folge hätte, können beispielsweise die Massen der bewegten Teile sowie die Auftreffgeschwindigkeit des Antriebsstücks derart eingestellt sein, daß die Stoßenergie im wesentlichen vollständig für das Ejizieren der Flüssigkeit aufgebraucht wird, so daß die zwischen dem Arbeitskolben und dessen Begrenzungswand vorliegende Reibung zum Aufrechterhalten der jeweiligen verlagerten Position des Arbeitskolbens ausreicht. Ferner kann auch in der Flüssigkeitsverbindung zwischen Druckkammer und Ejektionsöffnung ein Rückschlagventil vorgesehen sein, welches zwar das Ejizieren von Flüssigkeit erlaubt, jedoch das Zurücksaugen von Luft in die Druckkammer und damit ein Zurückstellen des Arbeitskolbens verhindert.

Bei dem erfindungsgemäßen Ejektionsgerät ist eine Linearfreilaufeinrichtung oder Rückverlagerungsstopeinrichtung vorgesehen, über die der Arbeitskolben gegen eine Zurückverlagerung abgestützt ist.

Die Linearfreilaufeinrichtung erlaubt es dem Arbeitskolben zwar in die Druckkammer hineinzuwandern, sie sperrt jedoch eine andernfalls von der teilweise am druckkammerseitigen Ende des Arbeitskolbens reflektierten Kompressionswelle verursachte Rückwärtsverlagerung des Arbeitskolbens. Dadurch bleibt der Arbeitskolben nach erfolgter Ejektion sicherer in derjenigen Position, die er am Ende des Ejektionsvorgangs eingenommen hat. Dadurch besteht auch keine Gefahr, daß Luft durch die Ejektionsöffnung in die Druckkammer eingesaugt wird. Zudem ist das Gerät nach jedem Stoß auch sofort wieder einsatzbereit. Ferner ist auch ein die Ejektionsöffnung beherrschendes Rückschlagventil in den meisten Fällen nicht erforderlich, da aufgrund der kontinuierlichen Vorwärtsbewegung des Arbeitskolbens die Gefahr eines Einsaugens von Luft durch die Ejektionsöffnung in die Druckkammer nicht besteht.

Die Anzahl der insgesamt möglichen Injektionen richtet sich danach, wie weit der Arbeitskolben in die Druckkammer wandern kann und wie groß der jeweilige Arbeitshub des Arbeitskolbens eingestellt ist. Auch beim letzten Arbeitshub soll aber das in der Druckkammer verbleibende Flüssigkeitsvolumen noch wesentlich größer sein als dieser Arbeitshub. Der Arbeitshub des Arbeitskolbens kann hierbei über die Stärke des von dem Antriebsstück übertragenen Stoßes eingestellt werden. Die pro Arbeitshub ejizierte Flüssigkeitsmenge liegt bevorzugt bei ca. 5-10 µl.

Die Linearfreilaufeinrichtung ist derart ausgelegt, daß sie die Verlagerung des Arbeitskolbens bei dessen Arbeitshub zuläßt, eine Zurückverlagerung des Arbeitskolbens jedoch verhindert. Die Freilaufeinrichtung kann hierbei nach dem Prinzip eines Reibgesperres ausgebildet sein, von dem Reibungskräfte ausgeübt werden, die bei der Vorwärtsverlagerung des Arbeitskolbens klein sind und überwunden werden, die sich aber bei einer versuchten Rückwärtsverlagerung des Arbeitskolbens derart erhöhen, daß eine solche unterbunden ist.

Ferner wäre als Freilaufeinrichtung auch ein den Arbeitskolben umgebender Ring aus Fasermaterial denkbar, dessen Fasern in Verlagerungsrichtung des Arbeitskolbens geneigt an diesem anliegen und sich bei einer Rückwärtsverlagerung des Arbeitskolbens ähnlich wie bei einem Schuppenski im Schnee unter Sperren dieser Verlagerung aufstellen.

Das erfindungsgemäße Ejektionsgerät ist insbesondere als medizinisches Gerät zur Ejektion von Medikamenten-Flüssigkeiten vorgesehen. Demnach muß das Risiko einer Kontamination der zu ejizierenden Flüssigkeit in der Druckkammer möglichst gering sein. Es ist daher wichtig, daß die Druckkammer effektiv gegen das Eindringen von Erregern abgedichtet ist. Auf der Ejektionsöffnung kann hierzu z.B. eine Verschlußkappe angeordnet sein. Arbeitskolbenseitig besteht jedoch das Problem, daß der Arbeitskolben mit dem bewegten Antriebsstück zur Stoßübertragung zusammenwirken muß und auch selbst verlagert wird. Hierbei besteht die Gefahr, daß durch das Zusammenwirken des Arbeitskolbens mit den nicht im adäquaten Maße sterilisierbaren antriebsseitigen Teilen Verunreinigungen und Erreger in die Druckkammer gelangen.

Dementgegenwirkend kann der Arbeitskolben beispielsweise mit einem einzelnen Dichtungselement, wie z.B. einem O-Ring oder einer Lippendichtung, gegen eine ihn umgebende Begrenzungswand umfangsseitig abgedichtet sein. Ein solches Dichtungselement hat auch den Vorteil, daß sich der beim Stoß auf die Flüssigkeit übertragene Hochdruckimpuls nicht in den Trennspalt zwischen dem Arbeitskolben und dessen zugeordneten Begrenzungswand nach hinten fortpflanzen kann, was zu einer Druckreduzierung in der Druckkammer und zu einem ungewollten Ausspritzen von Ejektionsflüssigkeit aus der Druckkammer nach hinten führen würde. Vorteilhafterweise sind an dem Arbeitskolben zwei in einem Abstand voneinander angeordnete elastische Dichtungselemente vorgesehen, von denen der Arbeitskolben umfangsseitig gegen eine zugehörige Begrenzungswand abgedichtet ist, wobei der Abstand der beiden Dichtungselemente größer ist als der Gesamtarbeitshub des Arbeitskolbens.

Dies hat den Vorteil, daß eine eventuell antriebsstückseitig auftretende Kontamination des Arbeitskolbens an dessen in Bewegungsrichtung des Arbeitskolbens gesehen vor dem druckkammerfernen Dichtungselement gelegenen Abschnitt nicht zusammen mit diesem Abschnitt über das druckkammernahe Dichtungselement hinweg in die Druckkammer hinein gelangen kann. Stattdessen gelangt ein derart kontaminierter Arbeitskolbenabschnitt lediglich zwischen die beiden Dichtungselemente, welche im übrigen im ausreichenden Maße ein Eindringen von Erregern verhindern.

Die den Arbeitskolben gegen seine Begrenzungswand abdichtenden Dichtungselemente sind insbesondere-während der beim Ejizieren der Flüssigkeit vorliegenden Hochdruckphase in der Druckkammer einer erhöhten Belastung ausgesetzt. Hierbei kann es evtl. passieren, daß sich die Druckwelle unter Überwindung der beiden Dichtungselemente in dem Trennspalt zwischen dem Arbeitskolben und dessen Begrenzungswand fortsetzt, was zu einem Austreten von Flüssigkeit aus der Druckkammer führen würde. Damit besteht die Gefahr, daß die Flüssigkeit zu den antriebsseitigen Teilen gelangt, welche hierdurch beschädigt werden können oder deren Funktion beeinträchtigt werden kann.

Um dies zu vermeiden, ist bevorzugt zwischen den beiden Dichtungselementen eine den Arbeitskolben umgebende, gasgefüllte Ringkammer ausgebildet. Überwindet die Flüssigkeit während der in der Druckkammer auftretenden Hochdruckphase das druckkammernahe Dichtungselement, dann verliert sie in der als Ausgleichsbehälter dienenden Ringkammer ihren hohen Druck und kann das zweite Dichtungselement nicht mehr passieren. Von der Ringkammer werden auch Kapillarkräfte in dem Spalt zwischen Arbeitskolben und diesen umgebenden Zylinder kompensiert.

Gemäß einer Ausführungsform ist zwischen dem Arbeitskolben und dem Antriebsstück ein den elastischen Stoß von dem Antriebsstück auf den Arbeitskolben übertragendes, von diesen separates Zwischenstück angeordnet.

Bei dieser erfindungsgemäßen Ausführungsform erfolgt die Stoßübertragung über das Zwischenstück, auf welches das Antriebsstück aufschlägt. Durch das Aufschlagen des Antriebsstücks auf das Zwischenstück, wird in diesem zunächst eine Stoßwelle angeregt, die sich in dem Zwischenstück fortpflanzt und von diesem auf den Arbeitskolben übertragen wird, wodurch der Arbeitskolben einen Hochdruckimpuls auf die Flüssigkeit in der Druckkammer überträgt. Das Vorsehen eines derartigen Zwischenstücks ermöglicht einige vorteilhafte Varianten des erfindungsgemäßen Ejektionsgeräts. So kann das Zwischenstück zur separaten Abdichtung des antriebsseitigen Teils des Ejektionsgeräts zur Druckkammer hin verwendet werden, z.B. zur Abdichtung einer Beschleunigungsstrecke, entlang welcher das Antriebsstück angetrieben wird, zur Druckkammer hin. Damit kann das oben als vorteilhaft erwähnte zweite Dichtungselement an dem Arbeitskolben ggf. entfallen, da für diesen die Gefahr einer Kontamination durch das Antriebsstück nicht mehr besteht.

Mit dem Zwischenstück besteht ferner die Möglichkeit, die Freilaufeinrichtung wahlweise an dem Arbeitskolben und/oder an dem Zwischenstück vorzusehen. Insbesondere im Fall, daß die Freilaufeinrichtung lediglich an dem Zwischenstück vorgesehen ist, kann der Arbeitskolben ausschließlich auf seine eigentliche, die Druckkammer begrenzende Kolbenfunktion hin ausgestaltet werden. Das Zwischenstück ist vorteilhafterweise aus einem festen Material, welches elastische Stoßwellen möglichst verlustfrei übertragen kann, wie z.B. einem Metallmaterial. Auch dieses Zwischenstück wirkt vorzugsweise durch bloße Übertragung von Kompressionswellen, die von dem elastischen Antriebsstoß ausgelöst werden. Das Zwischenstück ist ferner bevorzugt als Zwischenkolben vorgesehen, der durch einfache elastische Dichtungselemente, wie O-Ringe, umfangsseitig gegen eine entsprechende Begrenzungswand abgedichtet werden kann. Als Antriebsstück eignet sich in diesem Falle insbesondere ein Antriebskolben, der entlang eines Antriebsrohrs antreibbar ist, in das sich der Zwischenkolben abgedichtet hineinerstreckt. Der Zwischenkolben ist bevorzugt in Axialrichtung beidseitig durch einen Anschlag begrenzt, so daß er ungewollt weder aus dem Antriebsrohr noch zu weit in dieses gelangen kann.

Als eine an dem Zwischenkolben angreifende Freilaufeinrichtung kann neben den oben genannten Möglichkeiten beispielsweise auch eine Stopfbuchse vorgesehen sein, in die das vorlaufende Ende des Zwischenkolbens hineinwandern kann. Hierbei wäre die in Stoßrichtung verlaufende Kompressionswelle ausreichend stark, das vorlaufende Ende des Zwischenkolbens jeweils um den vorbestimmten Arbeitshub in die Stopfbuchse hinein auszulenken, wobei das ausgelenkte Ende dann in der Stopfbuchse stecken bleibt, so daß der restliche Zwischenkolben selbständig nachgeführt wird. Um das Einwandern des Zwischenkolbens in die Stopfbuchse zu erleichtern, ist das vorlaufende Ende des Zwischenkolbens vor dem Betrieb des Geräts vorteilhafterweise bereits in der Stopfbuchse angeordnet.

Besonders bevorzugt liegt das Zwischenstück spielfrei an dem Arbeitskolben an, so daß es nicht zu wesentlichen Energieverlusten durch eine anfängliche Beschleunigung und Bewegung des Zwischenstücks kommt. Dies bedeutet, daß das Zwischenstück zusammen mit dem Arbeitskolben mitwandern kann und selbst entsprechend verlagerbar gelagert ist. Das Mitwandern kann z.B. dadurch erreicht werden, daß die Freilaufeinrichtung an dem Zwischenstück oder an dem Zwischenstück und dem Arbeitskolben vorgesehen ist. Vorzugsweise sind jedoch der Arbeitskolben und das Zwischenstück über eine Kupplung lösbar aneinander befestigt.

Hierdurch wird auch ein geringfügiges Abheben des Arbeitskolbens von dem Zwischenstück unterbunden, was andernfalls zu einem periodischen Aneinanderschlagen der beiden Teile führen kann, was die nachfolgende Stoßübertragung beeinträchtigen würde. Bei dieser Ausführungsform kann die Linearfreilaufeinrichtung auch nur am Arbeitskolben angreifen.

Anstatt das Zwischenstück mit dem Arbeitskolben bei dessen Verlagerung mitzuführen, ist es auch möglich, das Zwischenstück nach der Verlagerung des Arbeitskolbens in Richtung auf diesen zu schrittweise axial nachzustellen. Bevorzugt ist hierzu das Zwischenstück von dem Antriebsstück während der Stoßübertragung auf den Arbeitskolben bis zum Anschlagen an einem Anschlagstück verschiebbar, das seinerseits in Richtung auf den Arbeitskolben zu schrittweise axial nachstellbar ist. Von dem Anschlagstück wird der Vorwärtshub des Zwischenstückes bei der Stoßübertragung begrenzt und bestimmt. Damit kann entsprechend die Dosiermenge der ejizierten Flüssigkeit bestimmt werden. Durch die Schrittverstellung des Anschlagstückes nach jeder Verlagerung des Arbeitskolbens kann somit erreicht werden, daß die Dosiermenge bei jeder der aufeinanderfolgenden Verlagerungen des Arbeitskolbens gleichbleibt. Es ist allerdings auch möglich, das Nachstellen des Anschlagstückes erst nach der Übertragung mehrerer Stöße nachzustellen.

Vorzugsweise wird das Anschlagen des Antriebsstücks auf das Anschlagstück reproduzierbar gedämpft, wodurch die Dosiergenauigkeit ebenfalls gesteigert werden kann. Ein einfaches Mittel hierzu ist die Anordnung eines elastischen Puffers mit definierter Federkennlinie zwischen dem Antriebsstück und dem Zwischenstück.

Das Nachstellen des Anschlagstückes kann beispielsweise mit Hilfe eines Gewindes erreicht werden, in das das Anschlagstück schraubverstellbar eingreift. Um hierbei die Dosiergenauigkeit nicht durch das unvermeidbare Gewindespiel zu beeinträchtigen, ist bevorzugt eine Feder vorgesehn, von welcher das Anschlagstück spielfrei an dem Gewinde abgestützt wird.

Zur Nachstellung des Anschlagstückes und - vorzugsweise auch - des Zwischenstückes kann beispielsweise das Antriebsrohr dienen, in welchem das Antriebsstück in Form eines Antriebskolbens hin- und herverschiebbar angeordnet ist und welches seinerseits entsprechend verstellbar ist.

Das Nachstellen des Zwischenstücks und/oder des Anschlagstückes kann manuell angetrieben erfolgen. Bevorzugt ist jedoch hierzu ein Schrittantrieb mit definierter, gegebenenfalls einstellbarer Schrittgröße vorgesehen.

Die Linearfreilaufeinrichtung wird von wenigstens einem elastischen Dichtungselement gebildet, von dem der Arbeitskolben oder das Zwischenstück umfangsseitig gegen die zugehörige Begrenzungswand abgedichtet ist.

Durch diese Doppelfunktion des Dichtungselements können einige zusätzliche Bauteile für die Linearfreilaufeinrichtung entfallen. Als Dichtungselement kann insbesondere eine Lippendichtung vorgesehen sein, deren Dichtungslippe in die Bewegungsrichtung des Arbeitskolbens geneigt ist. Wenn sich der Arbeitskolben entgegen der Druckkammer verlagern will, dann wird die derart geneigte Dichtungslippe aufgrund der Reibung zwischen ihr und dem Arbeitskolben entgegen ihrer Neigung mit nach hinten genommen und dadurch stärker gegen den Arbeitskolben gedrückt, wodurch dessen Zurückverlagerung verhindert wird. Wenn sich der Arbeitskolben in Richtung zu der Druckkammer verlagern will, erhöht sich der von der Dichtungslippe auf den Arbeitskolben ausgeübte Anpreßdruck dagegen fast nicht, so daß dem Arbeitskolben eine Verlagerung relativ zu der Dichtung ermöglicht ist. Eine solche Dichtungslippe wirkt demnach ähnlich wie der oben erläuterte Schwenkarm. Die sperrende Wirkung der Dichtungslippe kann noch dadurch verbessert werden, daß der Arbeitskolben mit einer leichten Riffelung versehen ist, z.B. mit in seiner Axialrichtung im Abstand voneinander angeordneten Umlaufkerben, deren Tiefe jedoch so gering ist, daß die Dichtungswirkung der Lippendichtung nicht beeinträchtigt ist. Es ist auch möglich, mehrere derartige Lippenringe als Packung zu verwenden. Eine andere Möglichkeit wäre auch, einen O-Ring unter einer derartigen Vorspannung zwischen dem Arbeitskolben und der zugehörigen Begrenzungswand oder zwischen einem kolbenförmigen Zwischenstück und einer zugehörigen Begrenzungswand anzuordnen, daß der gewünschte Linearfreilaufeffekt erreicht wird.

Bei Verwendung derartiger Dichtungsringe können ggf. gesonderte Dichtungen entfallen, wenn die Dichtungsringe gleichzeitig als Freilaufeinrichtung und als Abdichtungen eingesetzt und gestaltet werden.

Bei der Verwendung eines Zwischenstücks kann die Linearfreilaufeinrichtung vorteilhaft aufgeteilt von wenigstens zwei elastischen Dichtungselementen gebildet sein, von denen eines den Arbeitskolben und das andere das Zwischenstück umfangsseitig gegen die jeweils zugehörige Begrenzungswand abdichtet.

Wie aus.den bisherigen Ausführungen ersichtlich, ist das Zwischenstück bevorzugt auch zur Abdichtung des antriebsseitigen Geräteteils vorgesehen. Daher bietet es sich an, ein wie vorstehend beschriebenes Dichtungselement an dem Zwischenstück gleichzeitig auch als Freilaufteil einzusetzen, um zusätzliche Teile zu vermeiden. Entsprechendes gilt auch für das bevorzugt am Arbeitskolben vorgesehene Dichtungselement. Damit kann ein verbessert funktionsfähiger Linearfreilauf ohne Vorsehen von zusätzlichen Teilen erreicht werden.

Vorteilhafterweise kann der Freilauf außer Kraft gesetzt werden, um den Arbeitskolben bzw. das Zwischenstück nach erfolgter Wanderung des Arbeitskolbens in die Druckkammer entgegen des Freilaufs wieder in die ursprüngliche Position zurückstellen zu können. Im Falle der Anwendung eines Dichtungselements als Freilaufeinrichtung kann dieses beispielsweise als-bei eingesetztem Arbeitskolben bzw. Zwischenstück entnehmbar vorgesehen sein.

Gemäß einer bevorzugten Ausführungsform weist die Linearfreilaufeinrichtung als Dichtungselement einen O-Ring auf, der mittels eines den Arbeitskolben bzw. das Zwischenstück aufnehmenden, axial verstellbaren Klemmrings unter radialem Druck gegen den Arbeitskolben bzw. gegen das Zwischenstück in Axialrichtung zusammenpreßbar ist.

Dies hat den Vorteil, daß der Arbeitskolben bzw. das Zwischenstück zur Montage in einfacher Weise in den O-Ring einsetzbar bzw. zur Demontage aus diesem entnehmbar sind. Durch Verstellen des Klemmrings wird dann der Anpreßdruck des O-Rings bis zum Erreichen der gewünschten Freilaufeigenschaften erhöht. Damit es nicht dem Anwender überlassen ist, den richtigen Anpreßdruck einzustellen, ist für den Klemmring bevorzugt ein Anschlag vorgesehen, bis zu dem der Klemmring unter Zusammenpressen des O-Rings verstellt werden muß. Nachdem der Arbeitskolben die für ihn vorgesehene maximale Strecke in die Druckkammer gewandert ist, wird der Klemmring wieder gelöst, und der Arbeitskolben kann problemlos entnommen werden, um die Druckkammer z.B. zu reinigen, zu sterilisieren und ggf. nachzufüllen.

Bevorzugt weist die vorstehend erläuterte Linearfreilaufeinrichtung einen anderen O-Ring auf, der über eine benachbart zu dem Klemmring auf dem Arbeitskolben bzw. dem Zwischenstück angeordnete Distanzhülse in einem axialen Abstand von dem einen O-Ring angeordnet ist und der mittels des axial verstellbaren Klemmrings durch Kraftübertragung der Distanzhülse unter radialem Drücken gegen den Arbeitskolben bzw. das Zwischenstück in Axialrichtung zusammenpreßbar ist.

Diese Konstruktion ermöglicht die Ausbildung eines Linearfreilaufs mit, wie oben als vorteilhaft erläutert, zwei Dichtungselementen, welche entweder an dem Arbeitskolben oder an dem Zwischenstück angeordnet sind, wobei nur ein verstellbarer Klemmring vorzusehen ist. Diese Ausbildung der Freilaufeinrichtung eignet sich daher insbesondere für jene Ausführungsform, bei der kein Zwischenstück sowie zwei in Längsrichtung des Arbeitskolbens im Abstand voneinander angeordnete Dichtungselemente vorgesehen sind. Zudem kann auch die wie oben als vorteilhaft angegebene Ringkammer zwischen den beiden Dichtungselementen problemlos in der Distanzhülse vorgesehen werden.

Die wie vorstehend erläuterte Dichtanordnung mit dem Klemmring und dem/den von ihm axial zusammenpreßbaren O-Ring/en ist aber nicht lediglich bei gleichzeitiger Verwendung als Freilaufeinrichtung vorteilhaft, sondern ist auch dann als Dichtanordnung bevorzugt, insbesondere zur Abdichtung des Arbeitskolbens gegen dessen Begrenzungswand, wenn sie nicht die Freilaufeinrichtung bildet, da von ihr eine Fortpflanzung der Kompressionswelle in den Trennspalt zwischen dem Arbeitskolben und dessen Begrenzungswand besonders gut unterbunden wird

Obwohl das erfindungsgemäße Ejektionsgerät z.B. als integrales Einweggerät ausgebildet sein kann, bei dem beispielweise ein pneumatischer Antrieb mit einer in das Gerät integrierten Preßluftpatrone vorgesehen ist, die abgestimmt auf die maximale Anzahl an Ejektionen auf das Antriebsstück einwirkende Druckluft enthält, ist das Ejektionsgerät bevorzugt aufgeteilt in ein Gerätekopfteil, in dem der Arbeitskolben und zumindest teilweise die Druckkammer vorgesehen sind, und ein Geräteantriebsteil, in dem das Antriebsstück und ggf. das Zwischenstück vorgesehen sind. Hierbei sind das Gerätekopfteil und das Geräteantriebsteil als selbständige Geräteeinheiten ausgebildet, die über eine lösbare Kupplung aneinander befestigt sind.

Diese Konstruktion ermöglicht es, das Geräteantriebsteil als bleibendes Basisteil auszubilden und das Gerätekopfteil z.B. als austauschbares Einwegteil oder als abnehmbares Nachfüllteil vorzusehen. Bei der letzteren Variante kann das Gerätekopfteil einfach von dem Geräteantriebsteil abgenommen werden, der Kolben aus der Druckkammer entnommen werden und diese nachgefüllt werden. Die Kupplung zwischen dem Gerätekopfteil und dem Geräteantriebsteil kann z.B. als Schraub- oder Steckverbindung oder als Bajonettverschluß vorgesehen sein. Ein weiterer Vorteil der Aufteilung des Ejektionsgeräts in ein Gerätekopfteil und ein Geräteantriebsteil besteht ferner darin, daß das Geräteantriebsteil separat als ganzes in einem Sterilisationsgerät aufbereitbar, d.h. sterilisierbar ist.

Die Druckkammer kann beispielsweise lediglich in dem Gerätekopfteil vorgesehen sein, in welchem hierzu gleichzeitig auch die Ejektionsöffnung ausgebildet ist.

Bevorzugt weist die Druckkammer einen in dem Gerätekopfteil ausgebildeten Hohlraum auf, der wenigstens einen Teil der Druckkammer bildet und in dem gleichzeitig auch der Arbeitskolben aufgenommen ist. Der Hohlraum in dem Gerätkopfteil und der Arbeitskolben sind ferner vorteilhafterweise so ausgebildet, daß in der Endposition des Arbeitskolbens, d.h. wenn dieser bis zum Anschlagen an dem Boden des Hohlraums in die Druckkammer gewandert ist, nur eine geringe Restmenge an Flüssigkeit in dem Hohlraum verbleibt. Das bedeutet, daß der Boden des Hohlraums bevorzugt entsprechend dem ihm zugewandten Stirnende des Arbeitskolbens ausgebildet ist, so daß dieser mit seinem Stirnende formschlüssig am Boden des Hohlraums anliegt.

An dem Gerätekopfteil können eine Ejektionsdüse oder ein Endoskop-Katheter angebracht sein, die über eine Flüssigkeitsverbindung mit dem Hohlraum in Verbindung stehen. Der in der Ejektionsdüse oder in dem Endoskop-Katheter ausgebildete, flüssigkeitsführende Kanal bildet dann zusammen mit dem Hohlraum in dem Gerätekopfteil die Druckkammer aus. Der Endoskopkatheter kann als starrer oder als flexibler Katheter ausgebildet sein. Ferner kann der Endoskop-Katheter derart ausgebildet sein, daß er in den Arbeitskanal eines Endoskops einführbar ist. Hinsichtlich eines endoskopischen Einsatzes sind das Ejektionsgerät und das Endoskop auch bevorzugt zu einer Geräteeinheit zusammengefaßt.

Das Gerätekopfteil kann ferner mit einem Sichtfenster oder einer Anzeige versehen sein, die die Position des Arbeitskolbens anzeigt. Damit kann dann der aktuelle Füllstand der Druckkammer abgelesen werden.

Beim Zusammenbau des Gerätekopfteils mit dem Geräteantriebsteil muß bei vorgesehenem Zwischenstück gewährleistet sein, daß der Arbeitskolben und das Zwischenstück aneinander anliegen. Hierzu ist die Kupplung zwischen dem Arbeitskolben und dem Zwischenstück vorteilhafterweise fest an dem Zwischenstück vorgesehen und derart ausgebildet, daß sie beim Zusammenbau der beiden Geräteteile unter Schließen der Lücke zwischen Arbeitskolben und Zwischenstück automatisch mit dem Arbeitskolben verbunden wird. Im Falle daß ein kolbenförmiges Zwischenstück vorgesehen ist, ist die Kupplung vorteilhafterweise als Kupplungshülse ausgebildet, in die der Arbeitskolben und der Zwischenkolben einschnappen können. Beim Zusammenbau der beiden Geräteteile wird die Kupplungshülse vorab entweder an dem Zwischenkolben oder an dem Arbeitskolben festgelegt und kommt beim Zusammenfügen der Geräteteile nach und nach mit dem Arbeitskolben bzw. dem Zwischenkolben unter Herstellung der Verbindung zwischen denselben in Eingriff.

Die die Druckkammer begrenzenden Teile des Gerätekopfteils sind bevorzugt gut zu sterilisieren und steril zu halten. Hierzu weisen sie z.B. besonders glatte Außenflächen auf, in denen sich keine Verunreinigungen ansammeln können. Ferner müssen die Materialien dieser Teile auch inert gegenüber der zu ejizierenden Flüssigkeit sein, so daß diese die Teile nicht angreifen kann.

Im Falle daß das Gerätekopfteil als wiederauffüllbar vorgesehen ist, ist eine Befüllungsvorrichtung vorgesehen, mittels deren das Druckkammervolumen genau eingestellt werden kann, so daß die Druckkammer genau nachfüllbar ist. Die Befüllungsvorrichtung weist hierzu einen U-förmigen Rahmen auf, dessen einer Rahmenschenkel einen horizontalen Rahmensockel bildet und dessen anderer Rahmenschenkel ein horizontales Halteteil für das Gerätekopfteil bildet, wobei der Rahmensockel mit dem Halteteil über den vertikalen Steg des Rahmens verbunden ist. Das Gerätekopfteil kann von dem Halteteil derart festgehalten werden, daß der in dem Gerätekopfteil aufgenommene Arbeitskolben unter Einstellen des Druckkammervolumens vertikal bewegbar ist und mit seinem druckkammerfernen Stirnende an den Rahmensockel anstoßen kann. Der Rahmensockel bildet damit für den Arbeitskolben einen Anschlag, der das Druckkammervolumen genau festlegt. Die Flüssigkeit kann z.B. vor der genauen Einstellung des Druckkammervolumens in diese eingefüllt werden, um dann während des Einstellens teilweise wieder aus der Ejektionsöffnung herausgedrückt zu werden. Im Falle daß eine Ejektionsdüse oder ein Endoskop-Katheter an dem Gerätekopfteil angebracht ist, kann diese/dieser abgenommen werden, und die Flüssigkeit kann durch eine in dem Gerätekopfteil ausgebildete Flüssigkeitsaustrittsöffnung in den Hohlraum eingefüllt werden. Der Rahmen kann senkrecht zu seinem U-förmigen Querschnitt langgestreckt ausgebildet sein, so daß an dem Halteteil in dessen Längsrichtung in einem Abstand voneinander mehrere Gerätekopfteile gleichzeitig festgelegt und nachgefüllt werden können.

Diese Ausführungsform ist aber auch insbesondere dann vorteilhaft, wenn das Geräteantriebsteil mit einem Zwischenstück versehen ist. Das Zwischenstück wird vor dem Zusammenbau wieder in die Ausgangslage zurückgesetzt. Es kann durch dessen Ausgangsposition erreicht werden, daß beim Zusammenbau des Gerätekopfteils mit dem Geräteantriebsteil der Arbeitskolben von dem Zwischenstück etwas nach vorn gedrückt wird und entsprechend etwas Flüssigkeit aus der Druckkammer durch die Ejektionsöffnung hindurch verdrängt wird. Dadurch können eventuelle Gaseinschlüsse wie bei einer medizinischen Spritze vor dem Gebrauch ausgetrieben werden.

Nach einer bevorzugten Ausführungsform ist das Antriebsstück als pneumatisch antreibbarer Antriebskolben ausgebildet, der in einem Antriebsrohr angeordnet ist. Hierbei wird der Antriebskolben einfach mittels Druckluft aus seiner Ausgangsposition heraus in Richtung auf den Arbeitskolben beschleunigt. Zur Abführung der bei dieser Vorwärtsbewegung vom Arbeitskolben verdrängten Druckluft ist in dem Antriebsrohr an dessen arbeitskolbennahem Endabschnitt wenigstens ein Durchgangsloch ausgebildet, über welches der Innenraum des Antriebsrohrs mit einem Ausweichraum verbunden ist. Zur Rückführung des Antriebsstücks in seine Ausgangsposition wird die in den Ausweichraum abgeführte und dort komprimierte Luft benutzt, welche durch die Druchgangsöffnung zurück in das Innenrohr strömen kann und den Arbeitskolben in seine Ausgangsposition zurückdrückt. Hierzu ist das Durchgangsloch derart angeordnet, daß während des gesamten Betriebs des Ejektionsgeräts der Trennspalt zwischen dem Arbeitskolben und dem Antriebskolben auf Höhe der Lochfläche angeordnet ist. Da sich die Position dieses Trennspaltes durch die sukzessiv erfolgende Wanderbewegung des Arbeitskolbens ständig in Richtung zu der Druckkammer hin verlagert, ist das Durchgangsloch bervorzugt als Längsschlitz vorgesehen, dessen Länge sich nach dem erforderlichen Gesamtarbeitshub definiert. Nach einer bevorzugten Ausführungsform wird umgekehrt durch die Länge des Längsschlitzes, der Gesamtarbeitshub festgelegt. Hierzu wird ausgenutzt, daß die Luft nur solange aus dem Ausweichraum zurück in das Antriebsrohr strömen kann, bis der Antriebskolben mit seinem dem Arbeitskolben zugewandten Ende auf Höhe des dem Arbeitskolben zugewandten Endes des Längsschlitzes gewandert ist, so daß der Antriebskolben den Längsschlitz von innen her abdichtend abdeckt. Durch die Schlitzlänge wird somit die Wanderstreeke des Arbeitskolbens genau festgelegt.

Damit auch beim letzten Arbeitshub des Arbeitskolbens noch gewährleistet ist, daß die durch den bestimmten Verlagerungshub genau definierte Menge an Flüssigkeit ejiziert wird, ist bevorzugt der Hohlraums stets, d.h. auch beim letzten Arbeitshub noch größer als das Hubvolumen des Arbeitskolbens. Dies ist beispielsweise durch entsprechendes Festlegen der Länge des Längsschlitzes in einfacher Weise möglich. Es ist aber auch denkbar, den Arbeitskolben bis zum Anschlagen an der Hohlraumstirnseite in diese hineinwandern zu lassen und die Hohlraumlänge von vorneherein so auszulegen, daß auch beim letzten Arbeitshub, d.h. bis zum Anschlagen des Arbeitskolbens, noch'die korrekte Flüssigkeitsmenge ejiziert wird.

Um zu verhindern, daß das Antriebsstück seine Ausgangsposition ungewollt verläßt, ist eine Haltevorrichtung vorgesehen, mittels deren das Antriebsstück in der Ausgangsposition gehalten wird. Die Haltevorrichtung kann z.B. eine Klemmvorrichtung sein, mittels deren das Antriebsstück durch Klemmkräfte gehalten wird, welche von den Antriebskräften überwindbar sind. Vorteilhafterweise ist zum Halten des Antriebsstücks eine Magnethalterung vorgesehen, welche z.B. von einem Elektromagneten, bevorzugt aber von einem Dauermagneten gebildet ist. An dem Antriebsstück kann als Gegenstück zu der Magnethalterung ein magnetisches Teil, wie ein magnetisierbares Metallteil angebracht sein. Bevorzugt ist jedoch das gesamte Antriebsstück aus einem magnetisierbaren Werkstoff, wie z.B. Stahl.

Die Erfindung wird nachfolgend anhand bevorzugter Ausführungsformen mit Bezugnahme auf die Zeichnung erläutert. In der Zeichnung zeigen:
Figur 1 einen Längsschnitt eines Ejektionsgeräts zur Hochdruckejektion einer Flüssigkeit gemäß einer Ausführungsform der Erfindung,
Figur 2 eine vergrößerte Darstellung des vorderen Teils des in Figur 1 dargestellten Ejektionsgeräts,
Figur 3 einen Längsschnitt eines Ejektionsgeräts zur Hochdruckejektion einer Flüssigkeit gemäß einer anderen Ausführungsform der Erfindung,
Figur 4 eine vergrößerte Darstellung des vorderen Teils des in Figur 3 dargestellten Ejektionsgeräts,
Figur 5 eine Vorderansicht einer Befüllungsvorrichtung mit darin eingesetzten Gerätekopfteilen des erfindungsgemäßen Ejektionsgeräts,
Figur 6 einen Schnitt entlang der in Figur 5 mit A-A bezeichneten Linie,
Figur 7 einen dem in Figur 6 gezeigten Schnitt entsprechenden Schnitt einer erfindungsgemäßen Befüllungsvorrichtung,
Figur 8 einen Längsschnitt eines Ejektionsgeräts zur Hochdruckejektion einer Flüssigkeit gemäß einer anderen Ausführungsform der Erfindung,
Figur 9 eine vergrößerte Darstellung des vorderen Teils des in Figur 8 dargestellten Ejektionsgeräts, und
Fig. 10 einen Längsschnitt eines Ejektionsgerätes zur Hochdruck-Ejektion einer Flüssigkeit gemäß einer weiteren Ausführungsform der Erfindung.

In den Figuren sind gleiche Teile mit gleichen Bezugszeichen versehen.

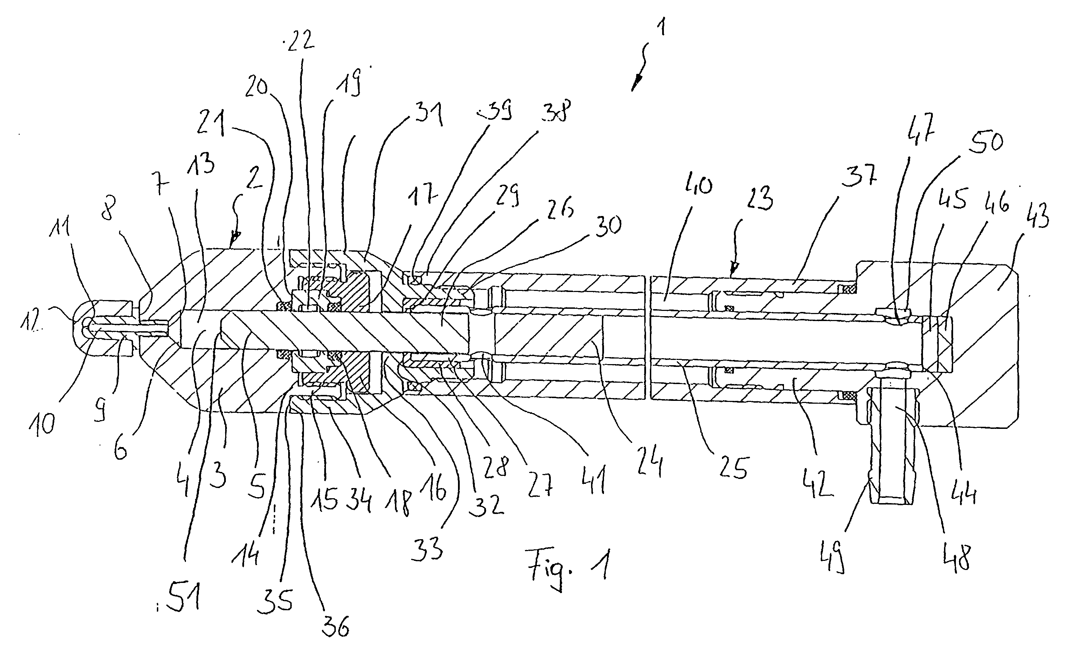

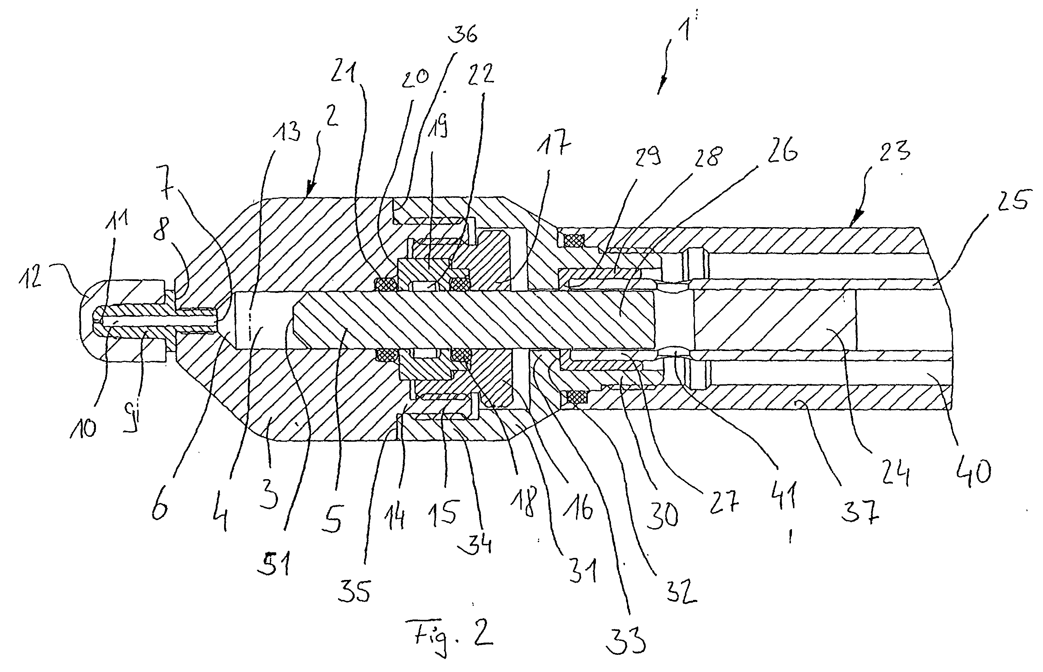

Figuren 1 und 2 zeigen ein erfindungsgemäßes Ejektionsgerät 1 zur Hochdruckejektion einer Flüssigkeit im Längsschnitt. Das Ejektionsgerät 1 weist ein Gerätekopfteil 2 mit einem Kopfteilkörper 3 auf, in dem ein kreiszylindrischer Hohlraum 4 ausgebildet ist, in dem seinerseits ein Arbeitskolben 5 in Axialrichtung beweglich aufgenommen ist. Der Hohlraum 4 ist an seinem arbeitskolbenfernen Endabschnitt 6 kegelförmig verjüngt und mündet in eine Hohlraumaustrittsöffnung 7. In den Kopfteilkörper 3 ist an dessen arbeitskolbenfernen Stirnende 8 eine Düse 9 in Form eines annähernd kreiszylindrischen, kurzen Rohrs eingeschraubt, in der ein Kanal 10 ausgebildet ist, der über die Hohlraumaustrittsöffnung 7 mit dem Hohlraum 4 in Verbindung steht und der in eine Ejektionsöffnung 11 mündet. Zum Erzielen des Düseneffekts weist der Kanal 10 benachbart zu der Ejektionsöffnung 11 einen kleineren Durchmesser auf als in seinem restlichen Abschnitt. Die Ejektionsöffnung 11 hat bevorzugt einen Durchmesser von 0,3 mm. Auf die Düse 9 ist eine die Ejektionsöffnung 11 verschließende Verschlußkappe 12 aufgesteckt. Der in dem Kopfteilkörper 3 ausgebildete Hohlraum 4 und der Kanal 10 in der Düse 9 bilden eine Druckkammer 13 aus, welche axial von dem Arbeitskolben 5 begrenzt ist.

An dem von der Düse 9 abgewandten Endabschnitt 14 des Kopfteilkörpers 3 ist ein Ringflansch 15 ausgebildet, der mit einem axial verlaufenden Außengewinde und einem zu diesem koaxialen Innengewinde versehen ist. In den Ringflansch 15 ist ein ein entsprechendes Außengewinde aufweisender, den Arbeitskolben 5 umgebender Klemmring 16 geschraubt, der einen nach innen weisenden Ringbund 17 aufweist, mit dem er an der der Druckkammer 13 zugewandten Ringbundseite gegen einen O-Ring 18 drückt, der an dem Arbeitskolben 5 kolbenumfangsseitig angeordnet ist. Dieser O-Ring 18 ist in einer inneren Umfangsnut einer auf dem Arbeitskolben 5 angeordneten Distanzhülse 19 aufgenommen, die ihrerseits in dem Klemmring 16 aufgenommen ist. Der Klemmring 16 drückt mit seinem nach innen weisenden Ringbund 17 auch die Distanzhülse 19 axial in Richtung zu der Druckkammer 13, und zwar sowohl gegen eine innere umlaufende Schulter 20 des Körpers 3, als auch gegen einen anderen an dem Arbeitskolben 5 umfangsseitig angeordneten O-Ring 21, welcher in einer in dem Kopfteilkörper 3 ausgebildeten inneren Ringnut aufgenommen ist, die zu der inneren Schulter 20 benachbart ist. Durch axiales Schraubverstellen des Klemmrings 16 in Richtung zu den O-Ringen 18, 21 wird der von den O-Ringen 18 und 21 jeweils auf den Arbeitskolben 5 ausgeübte radiale Preßdruck erhöht. Der Klemmring 16 ist hierbei solange verstellbar, bis die Distanzhülse 19 stirnseitig an der einen Anschlag bildenden inneren Schulter 20 des Kopf teilkörpers 3 anliegt. Der axiale Verstellweg des Klemmrings 16 ist derart gewählt, daß bei Anliegen der Distanzhülse 19 an der Schulter 20 die O-Ringe 18, 21 bezüglich des Arbeitskolbens 5 eine wie später erläuterte Linearfreilaufwirkung haben. Die Distanzhülse 19 ist zwischen den beiden O-Ringen 18, 21 mit einer inneren, d.h. zu dem Arbeitskolben 5 hin offenen, Ringnut 22 versehen, von der somit zwischen den beiden O-Ringen 18, 21 eine den Arbeitskolben 5 umgebende Ringkammer definiert wird.

Die an dem Kopfteilkörper 3 vorgesehene Dichtanordnung mit dem Klemmring 16, der Distanzhülse 19 und den O-Ringen 18 und 21 gehört ebenfalls zu dem Gerätekopfteil 2, welches, wie nachfolgend beschrieben, an einem Geräteantriebsteil 23 lösbar befestigt ist, von dem der Arbeitskolben 5 antreibbar ist.

Das Geräteantriebsteil 23 weist ein Antriebsstück in Form eines kreiszylindrischen Antriebskolbens 24 auf, der in einem kreiszylindrischen Antriebsrohr 25 längs frei bewegbar angeordnet ist. Das Antriebsrohr 25 und der darin aufgenommene Antriebskolben 24 sind zu dem Arbeitskolben 5 koaxial angeordnet, welcher sich seinerseits mit seinem druckkammerfernen Endabschnitt 26 in das Antriebsrohr 25 erstreckt. Auf den der Druckkammer 13 zugewandten Endabschnitt 27 des Antriebsrohrs 25, ist eine Stopfbuchse 28 aufgesteckt, die mit einem nach innen weisenden Ringbund 29 an dem Antriebsrohr 25 anliegt. Die Stopfbuchse 28 ist ihrerseits in einem von dem Kopfteilkörper 3 abgewandten Endabschnitt 30 einer ebenfalls zu dem Geräteantriebsteil 21 gehörenden Aufnahmemuffe 31 aufgenommen und liegt mit ihrem dem Kopfteilkörper 3 zugewandten Stirnende 32 an einem nach innen weisenden Ringbund 33 der Aufnahmemuffe 31 an. Die Aufnahmemuffe 31 weist an ihrem dem Kopfteilkörper 3 zugewandten Endabschnitt 34 ein axiales Innengewinde auf und ist mit diesem auf den Ringflansch 15 des Kopfteilkörpers 3 geschraubt. Das der Druckkammer 13 zugewandte Stirnende 35 der Aufnahmemuffe 31 liegt an einer Schulter 36 des Körpers 3 umfangsseitig bündig an.

Die lösbare Verbindung zwischen dem Gerätekopfteil 2 und dem Geräteantriebsteil 23 wird somit von einer zwischen dem an dem Kopfteilkörper 3 ausgebildeten Ringflansch 15 und der Aufnahmemuffe 31 vorliegenden Schraubverbindung gebildet.

An dem von dem Kopfteilkörper 3 abgewandten Endabschnitt 30 der Aufnahmemuffe 31 ist ein axiales Außengewinde ausgebildet. Ein das Antriebsrohr 25 aufnehmendes Gehäuse in Form eines kreiszylindrischen Aufnahmerohrs 37 ist an seinem dem Kopfteilkörper 3 zugewandten Endabschnitt 38 mit einem axialen Innengewinde versehen und auf den von dem Kopfteilkörper 3 abgewandten Endabschnitt 30 der Aufnahmemuffe 31 über einen O-Ring 39 umfangsseitig abgedichtet aufgeschraubt.

Zwischen dem Aufnahmerohr 37 und dem Antriebsrohr 25 ist eine Ringkammer 40 ausgebildet, die über benachbart zu der Stopfbuchse 28 in dem Antriebsrohr 25 ausgebildete, seitliche Öffnungen 41 mit dem Inneren des Antriebsrohrs 25 in Verbindung steht.

Die Ringkammer 40 wird an ihrem von dem Gerätekopfteil 2 abgewandten Ende axial von einer Dichthülse 42 begrenzt, die das Antriebsrohr 25 umgebend axial in das Aufnahmerohr 37 geschraubt ist und mittels O-Ringen 39, 40 gegen das Antriebsrohr 25 und das Aufnahmerohr 37 abgedichtet ist. An der Dichthülse 42 ist einstückig ein Stopfen 43 ausgebildet, von dem sowohl das von dem Kopfteilkörper 3 abgewandte Ende des Aufnahmerohrs 37 als auch das von dem Kopfteilkörper 3 abgewandte Ende des Antriebsrohrs 25 dicht verschlossen sind. Zwischen dem Stirnende 44 des Antriebsrohrs 25 und dem Stopfen 43 sind eine starre Platte 45 und eine elastische Platte 46 angeordnet, welche einen gerätekopfteilfernen, gepufferten Anschlag für den Antriebskolben 24 bilden. Die beiden Platten 45, 46 sind hintereinander angeordnet, wobei die starre Platte 45 dem Arbeitskolben 5 näher liegt. Vorteilhafterweise weisen die Platte 45 oder die Platte 46 magnetische Eigenschaften auf, und der Arbeitskolben ist aus einem magnetisierbaren Material, wie z.B. einem Stahlmaterial, so daß er in seiner Ausgangsposition an der Platte 45 über Magnetkräfte sicher gehalten ist und nicht ungewollt aus dieser Ausgangsposition gelangt. Um den Betrieb des Ejektionsgeräts zu gewährleisten, sind die Magnetkräfte jedoch kleiner als die Antriebskräfte gewählt. Benachbart zu dem vom Stopfen 43 verschlossenen Ende des Antriebsrohrs 25 sind in diesem seitliche Öffnungen 47 ausgebildet. In dem Stopfen 43 ist seitlich in Höhe der Öffnungen 47 ein Durchgangsloch 48 mit Innengewinde ausgebildet, in das ein Anschlußstutzen 49 für die Zuführung von Druckluft geschraubt ist. Das Durchgangsloch 48 steht über einen in dem Stopfen 43 ausgebildeten und das Antriebsrohr 25 in Höhe der Öffnungen 47 umgebenden Ringkanal 50 mit den Öffnungen 47 in Verbindung.

Im folgenden wird die Funktionsweise des vorstehend beschriebenen Ejektionsgeräts 1 beschrieben. Über eine nicht dargestellte Pumpe wird dem Ejektionsgerät 1 über den Anschlußstutzen 49 Druckluft zugeführt, die über den Ringkanal 50 und die Öffnungen 47 in das Innere des Antriebsrohrs 25 geleitet wird, wo sie auf den Antriebskolben 24 trifft. Dieser wird durch die zugeführte Preßluft in Richtung zu dem Arbeitskolben 5 beschleunigt, wobei die von dem Antriebskolben 24 in dem Antriebsrohr 25 verdrängte Luft durch die Öffnungen 41 in die Ringkammer 40 gepreßt wird. Der Antriebskolben 24 wird nur bis zum Anschlagen an dem Arbeitskolben 5 beschleunigt, d.h. von einer durch die zugeführte Preßluft bewirkten äußeren Kraft angetrieben. Anschließend wird der Antriebskolben 5 von der in der Ringkammer 40 komprimierten Luft, die über die Öffnungen 41 wieder zurück in das Antriebsrohr 25 strömt, zurückbewegt.

Mit dem Anschlagen des Antriebskolbens 24 auf den Arbeitskolben 5 wird in diesem eine Kompressionswelle angeregt, die den Arbeitskolben 5 durchläuft und eine Auslenkung des der Druckkammer 13 zugewandten Endes des Arbeitskolben 5 in den Hohlraum 4 hinein bewirkt. Dadurch erhöht sich der Druck in der Druckkammer 13 derart, daß die darin aufgenommene Flüssigkeit mit sehr hoher Geschwindigkeit aus der Ejektionsöffnung 11 ausgespritzt wird.

Die an dem Arbeitskolben 5 umfangsseitig vorgesehenen O-Ringe 18 und 21, von denen die Druckkammer 13 arbeitskolbenseitig abgedichtet ist, sind mittels des schraubverstellbaren Klemmrings 16 derart gegen den Arbeitskolben 5 gepreßt, daß dieser sich zwar durch den von dem Antriebskolben 24 verursachten Stoß in Figur 1 nach links in die Druckkammer 13, d.h. in den Hohlraum 4 verlagern kann, sich aber aufgrund der reflektierten, schwächeren Kompressionswelle nicht mehr aus der Druckkammer 13, d.h. aus dem Hohlraum 4, zurückverlagern kann. Damit wandert der Arbeitskolben 5 bei jedem Stoß des Antriebskolbens 24 auf den Arbeitskolben 5 unter Ejizieren einer kleinen Flüssigkeitsmenge ein Stück nach links in die Druckkammer 13 und verharrt aufgrund des über die O-Ringe 18, 21 erzielten Linearfreilaufs bis zum nächsten Auftreffen des Antriebskolbens 24 in dieser Position. Die radial gegen den Arbeitskolben 5 gedrückten O-Ringe 18, 21 bilden somit eine Linearfreilaufeinrichtung, die nach dem Prinzip eines Reibgesperres arbeitet.

Damit möglichst die gesamte in dem Hohlraum 4 enthaltene Flüssigkeit ejiziert werden kann, ist der Arbeitskolben 5 an seinem dem Hohlraum 4 zugewandten Stirnende 51 entsprechend dem arbeitskolbenfernen Endabschnitt 6 des Hohlraums 4 ausgebildet, d.h. kegelförmig verjüngt, so daß er formschlüssig an dem Boden des Hohlraums 4 anliegen kann.

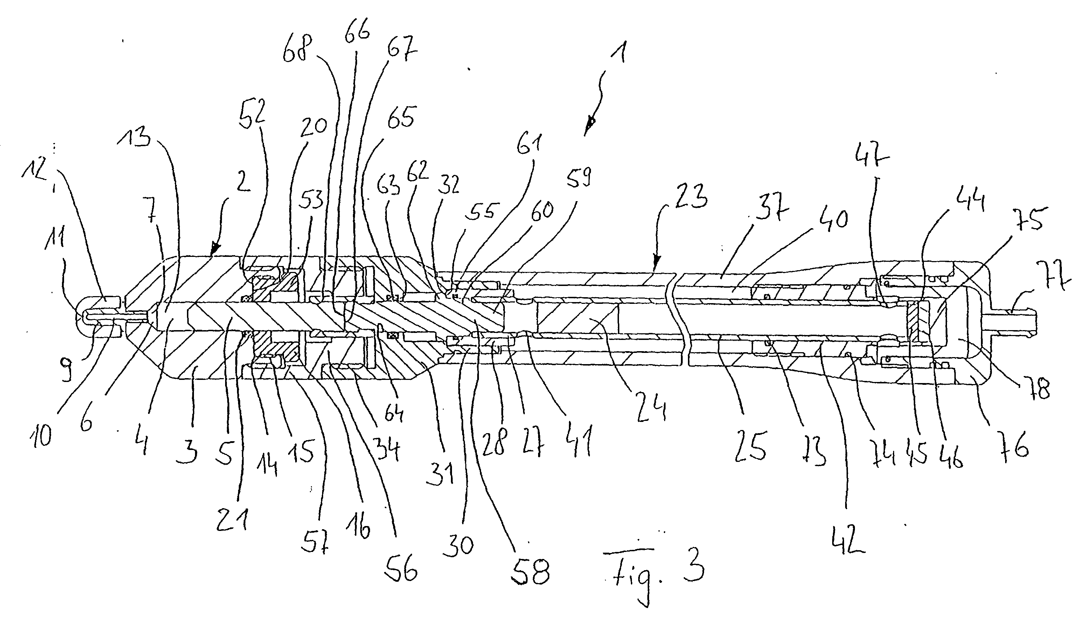

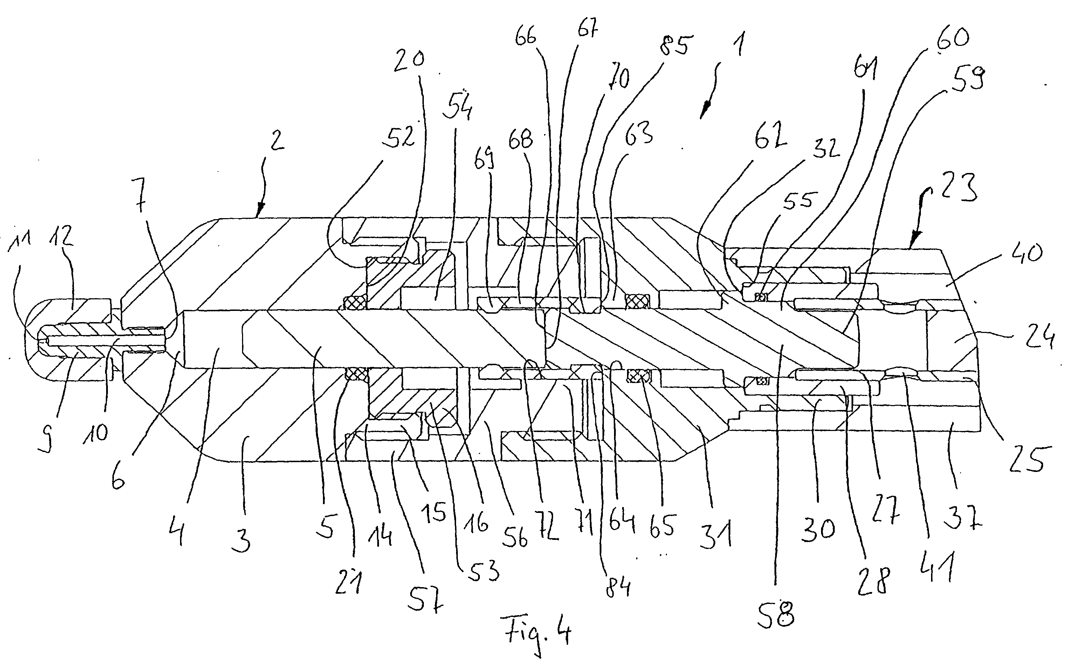

In Figuren 3 und 4 ist ein Ejektionsgerät 1 zur Hochdruckejektion einer Flüssigkeit gemäß einer anderen Ausführungsform der Erfindung gezeigt.

Das Ejektionsgerät 1 gemäß dieser Ausführungsform weist ein Gerätekopfteil 2 mit einem Kopfteilkörper 3 auf, in dem ein kreiszylindrischer Hohlraum 4 ausgebildet ist, in dem seinerseits ein den Hohlraum 4 axial begrenzender Arbeitskolben 5 in Axialrichtung beweglich aufgenommen ist. Der Hohlraum 4 ist an seinem arbeitskolbenfernen Endabschnitt 6 kegelförmig verjüngt und mündet in eine Hohlraumaustrittsöffnung 7. An dem Kopfteilkörper 3 ist, wie im Zusammenhang mit Figuren 1 und 2 erläutert, eine Düse 9 mit einer Ejektionsöffnung 11 vorgesehen, die von einer abnehmbaren Verschlußkappe 12 -verschlossen ist. Wie ferner im Zusammenhang mit Figuren 1 und 2 beschrieben, bilden der in dem Kopfteilkörper 3 ausgebildete Hohlraum 4 und ein in der Düse 9 ausgebildeter Kanal 10 eine Druckkammer 13 aus, welche axial von dem Arbeitskolben 5 begrenzt ist. An dem von der Düse 9 abgewandten Endabschnitt 14 des Kopfteilkörpers 3 ist ein Ringflansch 15 ausgebildet, der mit einem axial verlaufenden Außengewinde und einem zu diesem koaxialen Innengewinde versehen ist.

In den Ringflansch 15 ist ein ein entsprechendes Außengewinde aufweisender, den Arbeitskolben 5 umgebender Klemmring 16 geschraubt, der mit seinem der Druckkammer 13 zugewandten Stirnende 52 gegen eine innere, umlaufende Schulter 20 des Kopfteilkörpers 3 und einen an dem Arbeitskolben 5 umfangsseitig angeordneten O-Ring 21 drückt, welcher in einer in dem Kopfteilkörper 3 ausgebildeten inneren Ringnut aufgenommen ist, die benachbart zu der inneren Schulter 20 vorgesehen ist. Diese Dichtanordnung mit dem Klemmring 16 und dem O-Ring 21 gehört ebenfalls zu dem Gerätekopfteil 2.

Beim Einschrauben des Klemmrings 16 wird durch dessen axiales Schraubverstellen in Richtung zu dem O-Ring 21 der von dem O-Ring 21.auf den Arbeitskolben 5 ausgeübte radiale Preßdruck erhöht. Wie im Zusammenhang mit Figuren 1 und 2 erläutert, ist der maximale axiale Verstellweg des Klemmrings 16, d.h. der bis zum Anschlagen des Klemmrings 16 an der inneren Schulter 20 des Gerätekörpers 3 zurückgelegte Verstellweg, derart gewählt, daß der O-Ring 21 bezüglich des Arbeitskolbens 5 eine Linearfreilaufwirkung hat. Der Klemmring 16 ist an seinem von dem O-Ring 21 abgewandten Endabschnitt 53 unter Ausbildung einer den Arbeitskolben 5 umgebenden Ringkammer mit einer inneren, zu dem Arbeitskolben 5 hin offenen, ringförmigen Ausnehmung 54 versehen.

Das Ejektionsgerät 1 weist ferner ein Geräteantriebsteil 23 mit einem kreiszylindrischen Antriebskolben 24 auf, der in einem kreiszylindrischen Antriebsrohr 25 längs desselben frei bewegbar angeordnet ist. Das Geräteantriebsteil 23 und das Gerätekopfteil 2 sind, wie später noch genauer erläutert, lösbar aneinander befestigt. Das Antriebsrohr 25 und der darin aufgenommene Antriebskolben 24 sind zu dem Arbeitskolben 5 koaxial angeordnet. Auf den dem Kopfteilkörper 3 zugewandten Endabschnitt 27 des Antriebsrohrs 25 ist eine Stopfbuchse 28 aufgesteckt. Die Stopfbuchse 28 ist ihrerseits in einem von dem Kopfteilkörper 3 abgewandten Endabschnitt 30 einer zu dem Geräteantriebsteil 23 gehörenden Aufnahmemuffe 31 aufgenommen und liegt mit dem äußeren Umfangsrandabschnitt ihres dem Kopfteilkörper 3 zugewandten Stirnendes 32 an einer inneren Schulter 55 der Aufnahmemuffe 31 an. Die Aufnahmemuffe 31 weist an ihrem dem Kopfteilkörper 3 zugewandten Endabschnitt 34 ein axiales Innengewinde auf und ist mit diesem auf eine ein entsprechendes Außengewinde aufweisende Zwischenmuffe 56 abgedichtet aufgeschraubt, die ihrerseits an ihrem dem Kopfteilkörper 3 zugewandten Endabschnitt 57 mit einem Innengewinde versehen ist und mit diesem auf den Ringflansch 15 des Kopfteilkörpers 3 abgedichtet aufgeschraubt ist. Der Kopfteilkörper 3, die Aufnahmemuffe 31 und die Zwischenmuffe 56 sind zueinander koaxial angeordnet und grenzen umfangsseitig bündig aneinander an.

Die lösbare Kupplung zwischen dem Gerätekopfteil 2 und dem Geräteantriebsteil 23 wird somit von einer zwischen dem an dem Kopfteilkörper 3 ausgebildeten Ringflansch 15 und der zu dem Geräteantriebsteil 23 gehörenden Zwischenmuffe 56 vorliegenden Schraubverbindung gebildet.

Zu dem Geräteantriebsteil 23 gehört ferner ein teilweise in der Aufnahmemuffe 31 aufgenommenes Zwischenstück 58, das als im wesentlichen kreiszylindrischer Zwischenkolben ausgebildet ist und das zwischen dem Arbeitskolben 5 und dem Antriebskolben 24 sowie zu den beiden koaxial angeordnet ist. Das Zwischenstück 58 erstreckt sich mit seinem von der Druckkammer 13 abgewandten Endabschnitt 59 mit kleinem Radialspiel in das Antriebsrohr 25 und mit seinem zu diesem Endabschnitt 59 benachbarten Abschnitt 60, der einen relativ zu dem Endabschnitt 59 größeren Durchmesser hat, radial annähernd spielfrei in die Stopfbuchse 28. Zwischen dem Abschnitt 60 und der Stopfbuchse 28 ist ein rings des Zwischenstücks 58 verlaufender O-Ring 61 vorgesehen. An dem Zwischenstück 58 ist ein nach außen weisender Ringbund 62 ausgebildet, der bei voll mit Flüssigkeit gefüllter Druckkammer 13 auf seiner von dem Kopfteilkörper 3 abgewandten Seite an dem inneren Umfangsrandabschnitt des ihm zugewandten Stirnendes 32 der Stopfbuchse 28 anliegt. Die Aufnahmemuffe 31 hat einen nach innen weisenden Ringbund 63, von dem ein Durchgangsloch 64 ausgebildet ist, in dem das Zwischenstück 58 radial annähernd spielfrei und damit axial geführt aufgenommen ist. Der Ringbund 63 ist innen mit einer zu dem Zwischenstück 58 hin offenen Ringnut versehen, in der ein O-Ring 65 angeordnet ist, von dem das Zwischenstück 58 umfangsseitig gegen die Aufnahmemuffe 31 abgedichtet ist. Der nach innen weisende Ringbund 63 ist ausgehend von dem der Druckkammer 13 zugewandten Stirnende 32 der Stopfbuchse 28 in Richtung zu dem Kopfteilkörper 3 in einem axialen Abstand zu diesem Stirnende 32 angeordnet und bildet den druckkammernahen axialen Anschlag für den an dem Zwischenstück 58 vorgesehenen radialen Ringbund 62, welcher bei vollständig in den Hohlraum 4 gewandertem Arbeitskolben 5 an dem nach innen weisenden Ringbund 63 der Aufnahmemuffe 31 anliegt.

Das Zwischenstück 58 liegt mit seinem dem Arbeitskolben 5 zugewandten Stirnende 66 an dem ihm zugewandten Stirnende 67 des Arbeitskolbens 5 an und ist mit diesem über eine diese beiden Stirnenden 66, 67 umgebende Kupplungshülse 68 lösbar verbunden. Die Kupplungshülse 68 weist hierzu beidseitig zwei nach innen weisende Ringbünde 69, 70 auf (siehe Fig. 4), die in entsprechende Ringnuten in dem Arbeitskolben 5 und in dem Zwischenstück 58 eingreifen. Die Kupplungshülse 68 ist aus elastischem Material, so daß sie auf den Arbeitskolben 5 und auf das Zwischenstück 58 aufschnappen kann. Die Verriegelung zwischen dem Arbeitskolben 5 und der Kupplungshülse 68 ist durch eine radial nach innen verlaufende Verjüngung des zugehörigen Ringbunds 69 nicht so stark wie zwischen dem Zwischenstück 58 und der Kupplungshülse 68, da hier der zugehörige Ringbund 70 auf seiner dem Arbeitskolben 5 zugewandten Seite radial nach innen zumindest senkrecht zur Achse oder leicht schräg zu dem Arbeitskolben 5 hin unterschnitten verläuft, während die zugehörige Ringnut in dem Zwischenstück 58 radial nach außen senkrecht zur Achse oder von dem Arbeitskolben 5 schräg weg unterschnitten verläuft. Die Zwischenmuffe 56 hat einen nach innen weisenden Ringbund 71 (siehe Figur 4), von dem ein Durchgangsloch 72 ausgebildet wird, in dem die Kupplungshülse 68 radial annähernd spielfrei und damit axial geführt aufgenommen ist.

Auf den von dem Kopfteilkörper 3 abgewandten Endabschnitt 30 der Aufnahmemuffe 31 ist ein das Antriebsrohr 25 aufnehmendes, zu dem Geräteantriebsteil 23 gehörendes Gehäuse in Form eines kreiszylindrischen Aufnahmerohrs 37 abgedichtet aufgesteckt. Zwischen dem Aufnahmerohr 37 und dem Antriebsrohr 25 ist eine Ringkammer 40 ausgebildet, die über benachbart zu der Stopfbuchse 28 in dem Antriebsrohr 25 seitlich ausgebildete Öffnungen 41 mit dem Inneren des Antriebsrohrs 25 in Verbindung steht.

Die Ringkammer 40 wird an ihrem von dem Gerätekopfteil 2 abgewandten Ende axial von einer Dichthülse 42 begrenzt, die das Antriebsrohr 25 umgebend axial in das Aufnahmerohr 37 geschraubt ist und mittels O-Ringen 73, 74 gegen das Antriebsrohr 25 und das Aufnahmerohr 37 abgedichtet ist. Das von dem Gerätekopfteil 2 abgewandte Ende des Antriebsrohrs 25 ist mit einem Verschlußdeckel 75 verschlossen, der gleichzeitig einen hinteren Anschlag für den Antriebskolben 24 bildet. Zwischen dem Verschlußdeckel 75 und dem ihm zugewandten Stirnende 44 des Antriebsrohrs 25 sind eine starre und eine elastische Platte 45, 46 als Anschlag vorgesehen. Das gerätekopfteilferne Ende des Aufnahmerohrs 37 ist mit einem Stopfen 76 verschlossen, an dem ein Anschlußstutzen 77 für die Zuführung von Preßluft ausgebildet ist. Das Antriebsrohr 25 ist benachbart zu dem Verschlußdeckel 75 mit seitlichen Öffnungen 47 versehen, die über eine zwischen dem Stopfen 76, der Dichthülse 42, dem Aufnahmerohr 37 und dem Antriebsrohr 25 ausgebildete Kammer 78 mit der Öffnung des Anschlußstutzens 77 in Verbindung stehen.

Im folgenden wird die Funktionsweise des vorstehend beschriebenen Ejektionsgeräts 1 beschrieben. Der Antriebskolben 24 wird, wie im Zusammenhang mit Figuren 1 und 2 beschrieben, über Preßluft periodisch hin- und her angetrieben. Im Gegensatz zu der vorigen Ausführungsform stößt der Antriebskolben 24 jetzt gegen das Zwischenstück 58 und regt in diesem eine Kompressionswelle an, die von dem Zwischenstück 58 auf den Arbeitskolben 5 übertragen wird und eine Verlagerung dessen druckkammerseitigen Endes in die Druckkammer 13 hinein bewirkt. Dadurch wird, wie oben bereits erläutert, Flüssigkeit durch die Hohlraumaustrittsöffnung 7 und die Düse 9 aus der Ejektionsöffnung 11 ejiziert. Der an dem Arbeitskolben 5 vorgesehene O-Ring 21, von dem die Druckkammer 2 abgedichtet ist, ist mittels des schraubverstellbaren Klemmrings 16 radial derart gegen den Arbeitskolben 5 gepreßt, daß dieser sich zwar durch die von dem Antriebskolben 24 verursachte, in Stoßrichtung verlaufende Kompressionswelle in Figuren 3 und 4 nach links in die Druckkammer 13 verlagern kann, aber von der reflektierten, schwächeren Kompressionswelle nicht mehr aus der Druckkammer 2 zurückverlagerbar ist. Ferner ist der zwischen dem nach innen weisenden Ringbund 63 der Aufnahmemuffe 31 und dem Zwischenstück 58 angeordnete O-Ring 65 unter derartiger Vorspannung eingesetzt, daß er mit einem solchen Preßdruck radial gegen das Zwischenstück 58 drückt, daß dieses ebenfalls nur durch den Stoß in Richtung zu der Druckkammer 13 relativ zu dem O-Ring 65 verlagerbar ist.

Damit wandert der Arbeitskolben 5 bei jedem Auftreffen des Antriebskolbens 24 unter Ejizieren einer kleinen Flüssigkeitsmenge ein vorbestimmtes Stück in die Druckkammer 13 und verharrt aufgrund des über die O-Ringe 21 und 65 erzielten Freilaufs bis zum nächsten Auftreffen des Antriebskolbens 24 in seiner Position. Das an den Arbeitskolben 5 über die Kupplungshülse 68 angekuppelte Zwischenstück 58 wandert mit dem Arbeitskolben 5 mit.

Ist der Arbeitskolben 5 bis zum Anschlagen am in den Figuren 3, 4 linken Ende des Hohlraums 4 in die Druckkammer 13 gewandert, dann wird das Gerätekopfteil 2 abgenommen und nachgefüllt oder gegen ein anderes ausgetauscht. Das Gerätekopfteil 2 kann, wie aus den bisherigen Ausführungen ersichtlich, hierzu einfach von dem Geräteantriebsteil 23 abgeschraubt und, wie nachfolgend erläutert, gereinigt, sterilisiert und nachgefüllt oder gegen ein neues Gerätekopfteil 2 ausgetauscht werden.

Die Kupplungshülse 68 ist derart schwach mit dem Arbeitskolben 5 verbunden, daß sich dieser beim Lösen des Gerätekopfteils 2 von dem Geräteantriebsteil 23 von der Kupplungshülse 68 selbständig löst. Danach kann der Klemmring 16 aus dem Kopfteilkörper 3 geschraubt werden, wodurch auch der radiale Preßdruck des O-Rings 21 gegen den Arbeitskolben 5 aufgehoben wird. Dieser ist somit zusammen mit dem O-Ring 21 einfach aus dem Kopfteilkörper 3 entnehmbar. Desweiteren kann letztlich die Düse 9 von dem Kopfteilkörper 3 gelöst werden. Die Einzelteile des Gerätekopfteils 2 sind jetzt für Reinigungswerkzeuge gut zugänglich und können damit leicht gereinigt und sterilisiert werden.

Nach erfolgter Sterilisation werden der O-Ring 21 und der Arbeitskolben 5 wieder in den Kopfteilkörper 3 eingesetzt. Anschließend wird der Klemmring 16 eingeschraubt. Das Gerätekopfteil 2 kann jetzt z.B. durch die Hohlraumaustrittsöffnung 7 nachgefüllt werden, worauf die Düse 9 mit der Verschlußkappe 12 an dem Gerätekopfteil 2 angebracht werden. Das Gerätekopfteil 2 kann aber auch z.B. bei aufgesetzter Düse 9 und Verschlußkappe 12 über den noch nicht von dem Arbeitskolben 5 verschlossenen Hohlraum 4 nachgefüllt werden. In beiden Fällen kann der Arbeitskolben 5 nach dem Befüllvorgang ein Stück in die Druckkammer 13 bewegt werden, um evtl. darin vorliegende Luft aus der Ejektionsöffnung 11 auszudrücken.

Für den Zusammenbau der beiden Geräteteile 2, 23 wird das Zwischenstück 58 in seine Ausgangsposition, d.h. bis zum Anschlagen des Ringbundes 62 an dem Stirnende 32, zurückgeführt. Ist das Zwischenstück 58 in dieser Ausgangsposition, dann liegt das gerätekopfteilferne Stirnende 84 der Kupplungshülse 68 an einer dieser zugewandten inneren Schulter 85 der Aufnahmemuffe 31 an. Beim Anschrauben des Gerätekopfteils 2 an das Geräteantriebsteil 23 schiebt sich der Arbeitskolben 5 automatisch axial in die Kupplungshülse 68, welche aufgrund ihrer stirnseitigen Anlage an der als Anschlag dienenden inneren Schulter 84 der Aufnahmemuffe 31 nicht in die Einschraubrichtung des Gerätekopfteils 2 relativ zu dem Zwischenstück 58 bewegbar ist. Die Kupplungshülse 58 könnte jedoch auch derart an dem Zwischenstück 58 befestigt sein, daß zumindest eine axiale Relativbewegung zwischen Kupplungshülse 68 und Zwischenstück 58 ausgeschlossen ist.

Damit gewährleistet ist, daß der Arbeitskolben 5 und das Zwischenstück 58 aneinander anliegen, d.h. daß zwischen den beiden stirnseitig kein Hohlraum vorliegt, steht der Arbeitskolben 5 vor dem Zusammenbau des Gerätekopfteils 2 mit dem Geräteantriebsteil 23 bevorzugt wenigstens derart weit aus dem Hohlraum 4 in Richtung zu dem Geräteantriebsteil 23 vor, daß er an dem Zwischenstück anliegt, bevor das Gerätekopfteil 2 vollständig auf das Antreibsteil 23 aufgeschraubt ist. Dies wird durch ein entsprechendes Füllvolumen der Druckkammer 13 erreicht. Beim Aufschrauben des Gerätekopfteil 2 bleibt dann zunächst die Ejektionsöffnung 11 verschlossen, so daß sich der Arbeitskolben 5 beim Ankuppeln an das Zwischenstück 58 nicht unter Ejizieren von Flüssigkeit in die Druckkammer 13 bewegen kann. Ist der Arbeitskolben 5 mit dem Zwischenstück 58 verbunden, was daran feststellbar ist, daß sich das Gerätekopfteil 2 nicht weiter aufschrauben läßt, werden die Ejektionsöffnung 11 geöffnet und das Gerätekopfteil 2 vollständig auf das Geräteantriebsteil 23 aufgeschraubt. Hierbei wird in der Druckkammer 13 evtl. vorliegende Luft wie beim Entlüften einer Spritze ausgepreßt. Anschließend wird die Ejektionsöffnung 11 mit der Verschlußkappe 12 wieder verschlossen.

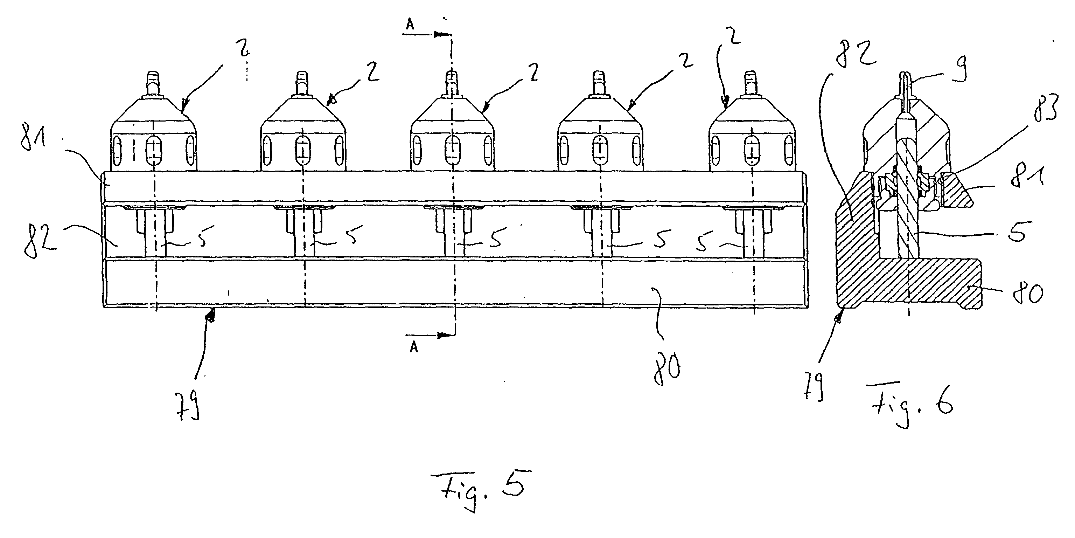

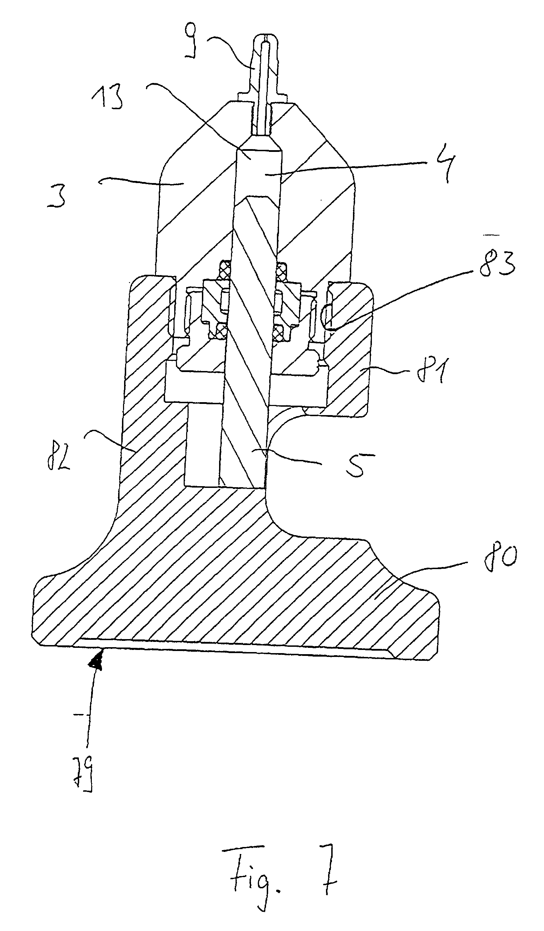

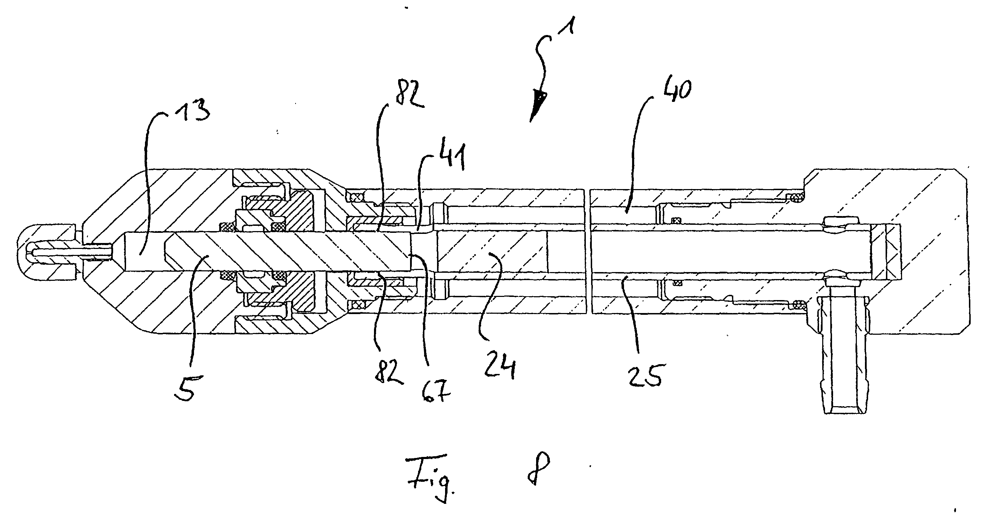

In Figuren 5 bis 7 ist eine Befüllungsvorrichtung dargestellt, mit der eines oder mehrere Gerätekopfteile 2 des erfindungsgemäßen Ejektionsgeräts 1 nachgefüllt werden können.

Die Befüllungsvorrichtung weist einen langgestreckten, im Querschnitt senkrecht zur Längsrichtung U-förmigen Rahmen 79 auf, dessen einer U-Schenkel einen horizontalen Rahmensockel 80 bildet und dessen anderer U-Schenkel ein horizontales Halteteil 81 bildet. Das Halteteil 81 und der Rahmensockel 80 sind über den U-Steg 82 des Rahmens 79 miteinander verbunden. In dem Halteteil 81 sind in Längsrichtung des Rahmens 79 in einem Abstand voneinander angeordnete Gewindelöcher 83 ausgebildet, in die jeweils ein Gerätekopfteil 2 mit seinem Kopfteilkörper 3, d.h. mit dem daran ausgebildeten Ringflansch 15, fest eingeschraubt ist. Der Arbeitskolben 5 des jeweils eingeschraubten Gerätekopfteils 2 verläuft vertikal und kann unter genauem Einstellen des Volumens des Hohlraums 4 in dem Kopf teilkörper 3 und damit unter genauem Einstellen des Druckkammervolumens an dem Rahmensockel 80 anstoßen. Das Halteteil 81 kann vertikal verstellbar vorgesehen sein, so daß unterschiedliche Druckkammervolumen einstellbar sind. Alternativ kann das Gerätekopfteil 2 unterschiedlich weit in das Halteteil 81 eingeschraubt werden, wodurch sich ebenfalls unterschiedliche Druckkammervolumen einstellen lassen. Hierzu ist bevorzugt an dem Rahmen 79 eine Anzeige vorgesehen, auf welcher die Einschraubtiefe des Kopfteilkörpers 3 ablesbar ist.

Zum Nachfüllen der Druckkammer 13 kann die Düse 9 abgenommen werden und die Flüssigkeit, z.B. mit einer Spritze, durch die Hohlraumaustrittsöffnung 7 nachgefüllt werden. Anschließend wird dann die Düse 9 mit ihrer Verschlußkappe 12 wieder an dem Gerätekopfteil angebracht. Die Flüssigkeit kann auch bevor der Arbeitskolben 5 in den Hohlraum 4 eingesetzt wird, direkt über diesen in die Druckkammer 13 eingefüllt werden. Um Lufteinschlüsse in der Druckkammer 13 zu vermeiden, kann es vorgesehen sein, das Gerätekopfteil 2 erst nach dem Befüllen vollständig in das Halteteil 81 einzuschrauben, so daß von dem Arbeitskolben 5 die evtl. in der Druckkammer 13 vorliegende Luft, wie beim Entlüften einer normalen Spritze, aus der Ejektionsöffnung 11 ausgedrückt wird. Anschließend wird die Verschlußkappe 12 auf die Düse 9 gesetzt.

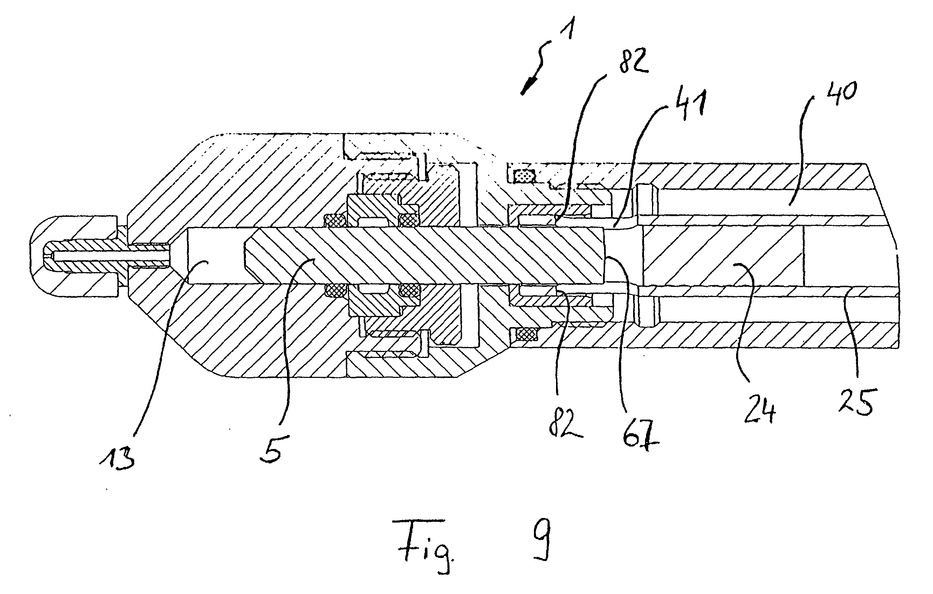

Figuren 8 und 9 zeigen ein erfindungsgemäßes Ejektionsgerät 1 zur Hochdruckejektion einer Flüssigkeit im Längsschnitt. Das Ejektionsgerät 1 entspricht dem in Figuren 1 und 2 dargestellten Ejektionsgerät 1 mit dem einen Zusatz, daß bei dieser Ausführungsform die seitlichen Öffnungen 41 als Längsschlitze ausgebildet sind. Durch das der Druckkammer 13 zugewandte Ende 82 der Längsschlitze ist die maximale Wanderstrecke des Arbeitskolbens 5, d.h. dessen Verlagerungsweg in die Druckkammer 13, definiert. Im einzelnen kann der Arbeitskolben 5 nur solange in die Druckkammer 13 hineinwandern, bis sein dem Antriebskolben 24 zugewandtes Ende 67 auf Höhe der Längsschlitzenden 82 ist, da dann der Antriebskolben 24 beim Anstoßen an dem Arbeitskolben 5 die Längsschlitze, deren Länge kleiner als die des Antriebskolbens 24 ist, komplett überdeckt, so daß die in der Ringkammer 40 komprimierte Luft nicht zurück in das Antriebsrohr 25 strömen kann, um den Antriebskolben 24 in seine Ausgangsposition zurückzuführen.

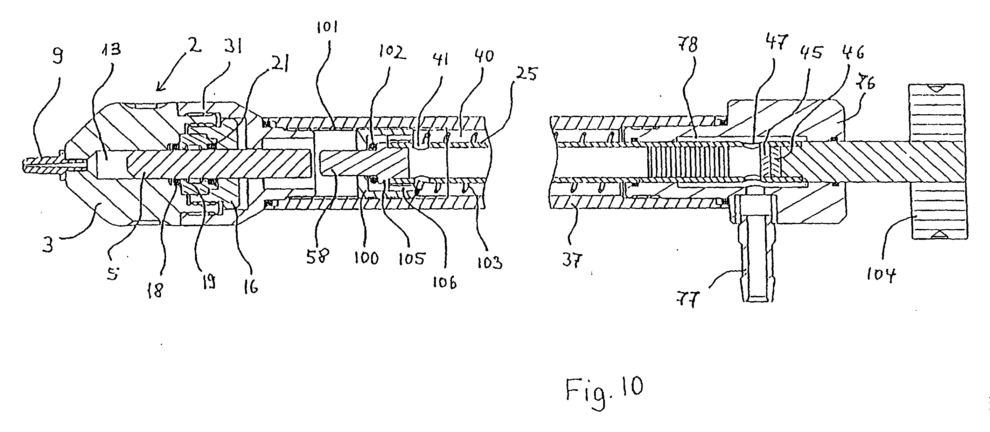

Die Ausführungsform eines erfindungsgemäßen Hochdruck-Ejektionsgerätes nach Fig. 10 entspricht vom Grundaufbau her derjenigen nach Fig. 3, so daß insoweit auf Fig. 3 verwiesen wird. Jedoch sind gemäß Fig. 10 der Zwischenkolben 58 und der Arbeitskolben 5 nicht miteinander gekuppelt, sondern unabhängig voneinander axial verstellbar. Außerdem kann der Zwischenkolben 58 jeweils nach der Übertragung des Stoßes auf den Arbeitskolben entsprechend dessen Verlagerung schrittweise nachgestellt werden.

Der Zwischenkolben 58 ist in einem topfartigen Anschlagstück 100 und in dem Antriebsrohr 25 verschiebbar geführt, das seinerseits in dem Gerätestopfen 76 drehbar und verschiebbar geführt ist. Das Anschlagstück 100 ist einstückig mit dem Antriebsrohr 25 fest gekuppelt und weist ein Außengewinde auf, das in ein Innengewinde 101 schraubverstellbar eingreift, welches in dem Aufnahmerohr 37 ausgebildet ist. Zwischen dem Stopfen 76 und dem Anschlagstück 100 ist eine Druckfeder 103 eingespannt, die in der Ringkammer 40 angeordnet ist und von der das Anschlagstück 100 an dem Gewinde 101 spielfrei abgestützt wird. Das Antriebsrohr 25 ist aus dem Gerätegehäuse axial herausgeführt und weist ein Einstellrad 104 zur Drehverstellung des Antriebsrohres und damit des Anschlagstückes 100 auf.

Der Zwischenkolben 58 weist einen radial nach außen ragenden Mitnehmerbund 105 auf, der zwischen die freie Stirnfläche des Antriebsrohres 25 und den Boden des Anschlagstücks 100 eingreift. Zwischen dem Mitnehmerbund 105 und dem Boden des Anschlagstücks 100 ist ein elastisch nachgiebiger Puffer 102 in Form eines Dämpfungsringes angeordnet. Im übrigen greift der Zwischenkolben 58 gleitend verschiebbar in das Antriebsrohr 25 einerseits und in den Boden des Anschlagstücks 100 andererseits ein. Dadurch kann der Zwischenkolben 58 durch Auftreffen des Antriebskolbens 24 in Richtung zu dem Arbeitskolben 5 gegen die Federkraft des Ringpuffers 102 um ein vorbestimmtes Maß verschoben werden, so daß auf den Arbeitskolben 5 ein definiert gedämpfter elastischer Stoß ausgeübt wird, der durch Einjustieren der Stellung des Anschlagstückes 100 und des Zwischenstückes eingestellt werden kann.

Im Kupplungssteg zwischen dem Ende des Antriebsrohres 25 und dem das Außengewinde aufweisenden Mantel des Anschlagstücks 100 sind Entlüftungslöcher 106 vorgesehen, so daß der der Zwischenkolben umgebende Raum innerhalb des topfförmigen Anschlagstückes 100 zu der Ringkammer 40 hin druckentlastet ist.

Für die folgende Beschreibung der Betriebsweise des Ejektionsgerätes nach Fig. 10 wird davon ausgegangen, daß der Arbeitskolben 5 bereits einen Verlagererungshub ausgeführt hat. Durch Drehen an dem Einstellräd 104 wird das Volumen des nachfolgenden Ejektionsschusses eingestellt, beispielsweise ein Volumen von 1,5 µl pro Drehung des Einstellrades 104 um 15°entsprechend einer Steigung des Vorschubgewindes 101 von 14xl. Die Drehbewegung wird von dem Einstellrad 104 über das Antriebsrohr 25 auf das Anschlagstück 100 übertragen, so daß das Anschlagstück 100 in dem Vorschubgewinde 101 des Aufnahmerohres 37 um ein entsprechendes axiales Maß in Richtung auf den Arbeitskolben 5 zu schraubend verstellt wird. Hierbei wird das Anschlagstück 100 von der Druckfeder 103 spielfrei gegen das Vorschubgewinde 101 in Richtung zu dem Arbeitskolben gedrückt. Das Antriebsrohr 37 wird um das gleiche axiale Maß mitgeführt und von dem Antriebsrohr 37 wird der Zwischenkolben 58 an den Mitnehmerbund um das gleiche axiale Maß nachgestellt.

Der Antriebskolben 24 wird nun durch impulsartige Druckluftzufur durch den seitlichen Anschlußstutzen 77 beschleunigt, wobei die verdrängte Luft durch die seitlichen Öffnungen 41 im Antriebsrohr 25 in die Ringkammer 40 verdrängt wird, trifft auf den Zwischenkolben 58 auf. Mithilfe der Entlüftungslöcher 106 wird der Druck hinter dem Mitnehmerbund 105 des Zwischenkolbens 58 ausgeglichen, so daß der Zwischenkolben nicht bereits vor dem Auftreffens des Antriebskolbens auf den Zwischenkolben druckbeaufschlagt wird. Durch das Auftreffen des Antriebskolbens 24 erhält der Zwischenkolben 58 einen Stoßimpuls und bewegt sich - aufgrund seiner geringeren Masse - schneller als der Antriebskolben 24 und leitet den Stoßimpuls solange an den Arbeitskolben 5 weiter, bis der Zwischenkolben auf das Anschlagstück 100 auftrifft. Dieses Auftreffen kann, wie im Ausführungsbeispiel nach Fig. 10 gezeigt, durch einen elastischen Puffer 102 oder durch eine andere Dämpfungsvorrichtung abgedämpft werden. Es ist jedoch auch möglich, den Puffer 102 oder die Dämpfungsvorrichtung wegzulassen, so daß das Zwischenstück 58 auf das Anschlagstück 100 ungedämpft auftrifft.

Das Nachstellen von Anschlagstück 100 und/oder Zwischenstück 58 kann manuell oder selbsttätig mithilfe eines Schrittantriebs erfolgen, der seinerseits manuell oder in Abhängigkeit von der Ejektionsfolge beziehungsweise von dem Antriebstakt des Antriebskolbens gesteuert aktiviert wird. Hierbei ist es auch möglich, einen vollautomatisch gesteuerten Schrittantrieb vorzusehen, der auf einen diskreten Zustand, beispielsweise auf den Druckanstieg in der Ringkammer, auf das Auftreffen des Antriebskolbens auf das Zwischenstück oder des Zwischenstücks auf den Arbeitskolbens oder auf das Zurückkehren des Antriebskolbens in seine Startstellung anspricht.

## Patentansprüche

1. Ejektionsgerät (1) zur Hochdruckejektion einer Flüssigkeit oder einer Partikel enthaltenden Flüssigkeit, mit einer die Flüssigkeit aufnehmenden Druckkammer (13), die in eine Ejektionsöffnung (11) ausmündet und die von einem Arbeitskolben (5) begrenzt wird, wobei von dem Arbeitskolben (5), durch Übertragung eines Stoßes auf das druckkammerferne Ende des Arbeitskolbens (5), eine Kompressionswelle übertragbar ist, durch welche wenigstens das druckkammerseitige Ende des Arbeitskolbens (5) in die Druckkammer (13) um einen vorbestimmten Verlagerungshub unter Reduzierung von deren Volumen auslenkbar ist, wobei die Volumenreduktion der Druckkammer (13) wesentlich kleiner ist als das Druckkammervolumen, und einem Antriebsstück (24), von dem der Stoß erzeugbar ist, wobei der Arbeitskolben (5) durch aufeinanderfolgend auf ihn ausgeübte Stöße sukzessive weiter jeweils um den vorbestimmten Verlagerungshub in die Druckkammer (13) hinein verlagerbar und über eine Linearfreilaufeinrichtung gegen eine Zurückverlagerung abgestützt ist, **dadurch gekennzeichnet, daß** die Linearfreilaufeinrichtung von wenigstens einem elastischen Dichtungselement (18; 21; 65) gebildet wird, welches den Arbeitskolben oder ein, der Stoßübertragung auf den Arbeitskolben dienendes, mögliches Zwischenstück (58) umfangsseitig gegen eine zugehörige Begrenzungswand abdichtet und der Zurückverlagerung des Arbeitskolbens (5) mehr Widerstand entgegensetzt als der Verlagerung des Arbeitskolbens (5) in die Druckkammer (13) hinein.

2. Ejektionsgerät (1) nach Anspruch 1, **dadurch gekennzeichnet, daß** an dem Arbeitskolben (5) zwei in einem Abstand voneinander angeordnete elastische Dichtungselemente (18, 21) vorgesehen sind, von denen der Arbeitskolben (5) umfangsseitig gegen eine zugehörige Begrenzungswand abgedichtet ist, und daß der Abstand der beiden Dichtungselemente (18, 21) größer ist als der Gesamtarbeitshub des Arbeitskolbens (5), wobei zwischen den beiden Dichtungselementen (18, 21) eine den Arbeitskolben (5) umgebende, gasgefüllte Ringkammer (22) ausgebildet ist.

3. Ejektionsgerät (1) nach einem der Ansprüche 1 oder 2, **dadurch gekennzeichnet, daß** zwischen dem Arbeitskolben (5) und dem Antriebsstück (24) ein den elastischen Stoß von dem Antriebsstück (24) auf den Arbeitskolben (5) übertragendes, von diesen separates Zwischenstück (58) angeordnet ist.

4. Ejektionsgerät (1) nach Anspruch 3, **dadurch gekennzeichnet, daß** der Arbeitskolben (5) und das Zwischenstück (58) über eine Kupplung (68) lösbar aneinander befestigt sind.

5. Ejektionsgerät (1) nach Anspruch 3 oder 4, **dadurch gekennzeichnet, daß** die Linearfreilaufeinrichtung an dem Arbeitskolben (5) und/oder an dem Zwischenstück (58) vorgesehen ist.

6. Ejektionsgerät (1) nach einem der Ansprüche 3 bis 5, **dadurch gekennzeichnet, daß** die Linearfreilaufeinrichtung von wenigstens zwei elastischen Dichtungselementen (18, 21; 65) gebildet ist, von denen eines den Arbeitskolben (5) und das andere das Zwischenstück (58) umfangsseitig gegen die jeweils zugehörige Begrenzungswand abdichtet.

7. Ejektionsgerät (1) nach einem der vorhergehenden Ansprüche, **dadurch gekennzeichnet, daß** die Linearfreilaufeinrichtung als Dichtungselement einen O-Ring (18, 21) aufweist, der mittels eines verstellbaren Klemmrings (16) unter radialem Druck gegen den Arbeitskolben (5) bzw. gegen das Zwischenstück (58) in seine Axialrichtung zusammenpreßbar ist.

8. Ejektionsgerät (1) nach Anspruch 7, **dadurch gekennzeichnet, daß** die Linearfreilaufeinrichtung einen anderen O-Ring (21) aufweist, der mittels einer benachbart zu dem verstellbaren Klemmring (16) angeordneten Distanzhülse (19) in einem axialen Abstand von dem einen O-Ring (18) angeordnet ist und der mittels des verstellbaren Klemmrings (16) durch Kraftübertragung der Distanzhülse (19) unter radialem Druck gegen den Arbeitskolben (5) bzw. gegen das Zwischenstück (58) in seiner Axialrichtung zusammenpreßbar ist.

9. Ejektionsgerät nach einem der Ansprüche 1 bis 8, **gekennzeichnet durch** ein Gerätekopfteil (2), in dem der Arbeitskolben (5) und zumindest teilweise die Druckkammer (13) vorgesehen sind, und ein Geräteantriebsteil (23), in dem das Antriebsstück (24) bzw. das Zwischenstück (58) vorgesehen sind, wobei das Gerätekopfteil (2) und das Geräteantriebsteil (23) als selbständige Geräteeinheiten ausgebildet sind, die über eine lösbare Kupplung aneinander befestigt sind.

10. Ejektionsgerät (1) nach Anspruch 9, **dadurch gekennzeichnet, daß** die Druckkammer (13) zumindest teilweise von einem in dem Gerätekopfteil (2) ausgebildeten Hohlraum (4) gebildet ist, in dem auch der Arbeitskolben (5) aufgenommen ist, wobei der Boden des Hohlraums (4) entsprechend dem ihm zugewandten Stirnende (51) des Arbeitskolbens (5) geformt ist.

11. Ejektionsgerät (1) nach Anspruch 9 oder 10, **dadurch gekennzeichnet, daß** das Gerätekopfteil (2) als austauschbares Einwegteil ausgebildet ist.

12. Ejektionsgerät (1) nach Anspruch 11 oder 12, **dadurch gekennzeichnet, daß** das Gerätekopfteil (2) als nachfüllbares Teil ausgebildet ist, welches zum Nachfüllen von dem Geräteantriebsteil (23) abnehmbar ist.

13. Ejektionsgerät (1) nach einem der Ansprüche 1 bis 12, **dadurch gekennzeichnet, daß** das Antriebsstück (24) als pneumatisch antreibbarer Antriebskolben (24) ausgebildet ist, der in einem Antriebsrohr (25) angeordnet ist, welches an seinem dem Arbeitskolben (5) zugewandten Endabschnitt einen Längsschlitz aufweist, über den der Innenraum des Antriebsrohrs (24) mit einem Ausweichraum (40) in Verbindung steht, wobei der Antriebskolben (24) von der in den Ausweichraum (40) durch den Längsschlitz hindurch abgeführten und komprimierten Luft solange wieder in seine Ausgangsposition zurückbewegbar ist, bis der Antriebskolben (24) das dem Arbeitskolben (5) zugewandte Ende des Längsschlitzes erreicht.

14. Ejektionsgerät (1) nach einem der Ansprüche 1 bis 13, **gekennzeichnet durch** einen Endoskop-Katheter mit einem flüssigkeitsführenden Kanal, von dem die Druckkammer (13) teilweise ausgebildet ist.

15. Ejektionsgerät nach Anspruch 3, **dadurch gekennzeichnet, daß** das den Stoß von dem Antriebsstück (24) auf den Arbeitskolben (5) übertragende Zwischenstück (58) nach der Stoßübertragung in Richtung auf den Arbeitskolben (5) zu axial nachstellbar ist.

16. Ejektionsgerät nach Anspruch 15, **dadurch gekennzeichnet, daß** das Zwischenstück (58) von dem Antriebsstück (24) in Richtung auf den Arbeitskolben (5) zu bis zum Anschlagen an einem Anschlagstück (100) verschiebbar ist, das seinerseits in Richtung auf den Arbeitskolben (5) axial nachstellbar ist.

17. Ejektionsgerät nach Anspruch 16, **dadurch gekennzeichnet, daß** zwischen dem Anschlagstück (100) und dem Zwischenstück (58) ein das Anschlagen des Zwischenstücks elastisch dämpfender Puffer (102) angeordnet ist.

18. Ejektionsgerät nach Anspruch 16 oder 17, **dadurch gekennzeichnet, daß** das Anschlagstück (100) mittels eines Gewindes (101) axial nachstellbar ist und an dem Gewinde von einer Feder (103) spielfrei abgestützt ist.

19. Ejektionsgerät nach einem der Ansprüche 16 bis 18, **dadurch gekennzeichnet, daß** das Anschlagstück (100) und das Zwischenstück (58) mittels eines Antriebsrohr (25) nachverstellbar sind, in dem das Antriebsstück (24) angeordnet ist und das verstellbar geführt ist.

20. Ejektionsgerät nach einem der Ansprüche 16 bis 19, **dadurch gekennzeichnet, daß** ein das Anschlagstück (100) nachstellender Schrittantrieb vorgesehen ist.

## Claims

1. An ejection device (1) for the high-pressure ejection of a liquid or a liquid containing solid particles, comprising a pressure chamber (13) wherein the liquid is received, which opens into an ejection opening (11) and is delimited by a working piston (5) which, upon application of an impact on its end facing away from the pressure chamber, is capable of transmitting a compression wave by which at least the pressure chamber-facing end of the working piston (5) is displaceable by a predetermined displacement stroke into the pressure chamber (13) so that the volume thereof is reduced, with the reduction in volume of the pressure chamber (13) being significantly smaller than the volume of the pressure chamber (13); and a drive member (24) which is capable of generating the impact, wherein the working piston (5) is successively displaced into the pressure chamber (13) by the repeated application of impacts, with the distance of each single displacement being defined by the predetermined displacement stroke, and supported by a linear reverse stopping means so that a reverse displacement thereof is prevented, **characterized in that** the linear reverse stopping means is formed by at least one elastic sealing element (18; 21; 65) which seals the circumference of the working piston or of a possible intermediary member (58) serving the transmission of the impact to the working piston against an associated wall member and which resists the reverse displacement of the working piston (5) more than the displacement of the working piston (5) into the pressure chamber (13).

2. An ejection device (1) according to claim 1, **characterized in that** the working piston (5) is provided with two elastic sealing elements (18, 21) arranged at a distance from each other to seal the circumference of the working piston (5) against an associated wall member, and that said distance between the two sealing elements (18, 21) is greater than the total working stroke of the working piston (5), wherein a gas-filled annular chamber (22) surrounding the working piston (5) is formed between the two sealing elements (18, 21).

3. An ejection device (1) according to claim 1 or 2, **characterized in that** an intermediary member (58) is provided as a separate component between the working piston (5) and the drive member (24), which transmits the elastic impact from the drive member (24) to the working piston (5).

4. An ejection device (1) according to claim 3, **characterized in that** the working piston (5) and the intermediary member (58) are separably connected to each other by means of a coupling (68).

5. An ejection device (1) according to claim 3 or 4, **characterized in that** the linear reverse stopping means is provided at the working piston (5) and/or at the intermediary member (58).

6. An ejection device (1) according to any one of the claims 3 to 5, **characterized in that** the linear reverse stopping means is formed by at least two elastic sealing elements (18, 21; 65), each of which seal the circumference of one of the working piston (5) and the intermediary member (58) against their associated wall member.

7. An ejection device (1) according to any one of the above claims, **characterized in that** the linear reverse stopping means comprises an O-ring (18, 21) as a sealing element which is axially compressible by an adjustable clamping ring (16) so that a radial pressure is exerted on the working piston (5) and the intermediary member (58), respectively.

8. An ejection device (1) according to claim 7, **characterized in that** the linear reverse stopping means comprises another O-ring (21) which is axially spaced apart from the first O-ring (18) by a distance seal (19) arranged adjacent to the adjustable clamping ring (16), and which may be axially compressed by means of the adjustable clamping ring (16), with the distance sleeve (19) acting as a power transmitting member, so that a radial pressure is exerted on the working piston (5) and the intermediary member (58), respectively.

9. An ejection device (1) according to any one of the claims 1 to 8, **characterized by** a head unit (2) wherein the working piston (5) and at least a portion of the pressure chamber (13) are provided, and a drive unit (23) wherein the drive member (24), and the intermediary member (58), respectively, are provided with the head unit (2) and the drive unit (23) being formed as independent, separate units of the ejection device which are connected to each other by means of a separable coupling.

10. An ejection device (1) according to claim 9, **characterized in that** at least a portion of the pressure chamber (13) is formed by a cavity (4) provided in the head unit (2) and accommodating the working piston (5) as well, with the bottom of the cavity (4) being shaped such that it conforms with the opposing end face (51) of the working piston (5).

11. An ejection device (1) according to claim 9 or 10, **characterized in that** the head unit (2) is designed as an exchangeable disposable unit.

12. An ejection device (1) according to any one of the claims 11 to 12, **characterized in that** the head unit (2) is designed as a refillable unit which is removable from the drive unit (23) to be refilled.

13. An ejection device (1) according to any one of the claims 1 to 12, **characterized in that** the drive member (24) is designed as a pneumatically driveable drive piston (24) which is arranged in a drive pipe (25) that comprises a longitudinal slot at its end portion facing towards the working piston (5), said longitudinal slot providing a fluid communication between the interior of the drive pipe (25) and a venting chamber (40), the drive piston (24) is returnable to its starting position by means of the pressure generated by the air forced through the longitudinal slot into the venting chamber (40) and compressed therein, until the drive piston (24) reaches the working piston-facing and of the longitudinal slot.

14. An ejection device (1) according to any one of the claims 1 to 13, **characterized by** an endoscopic catheter comprising a liquid-carrying passage which forms a portion of the pressure chamber (13).

15. An ejection device (1) according to claim 3, **characterized in that** the intermediary member (58) transmitting the impact from the drive member (24) to the working piston (5) may be axially adjusted towards the working piston (5) after the impact has been transmitted.

16. An ejection device (1) according to claim 15, **characterized in that** the intermediary member (58) is displaceable towards the working piston (5) by the drive member (24), until it abuts against a stopper member (100) which is axially adjustable towards the working piston (5).

17. An ejection device (1) according to claim 16, **characterized in that** a buffer (102) is arranged between the stopper member (100) and the intermediary member (58), which serves to elastically dampen the impact of the intermediary member.

18. An ejection device (1) according to claim 16 or 17, **characterized in that** the stepper member (100) is axially adjustable by means of a thread (101), and supported by a spring (103) so that it abuts at said thread without play.

19. An ejection device (1) according to any one of the claims 16 to 18, **characterized in that** the stopper member (100) and the intermediary member (58) are adjustable by means of a drive pipe (25) wherein the drive member (24) is arranged and which is displaceable guided.

20. An ejection device (1) according to any one of the claims 16 to 19, **characterized in that** a stepper drive is provided for adjusting the stopper member (100).

## Revendications

1. Appareil éjecteur (1) pour l'éjection à haute pression d'un liquide ou d'un liquide contenant des particules, avec une chambre de pression (13) recevant le liquide, chambre débouchant sur une ouverture d'éjection (11) et délimitée par un piston de travail (5), une onde de compression étant susceptible d'être transmise par le piston de travail (5) par transmission d'un choc sur l'extrémité, éloignée de la chambre de compression, du piston de travail (5), onde de compression au moyen de laquelle au moins l'extrémité située du côté de la chambre de pression du piston de travail (5) est susceptible d'être déplacée dans la chambre de pression (13) d'une course de déplacement prédéterminée accompagnée de la réduction de son volume, la réduction de volume de la chambre de pression (13) étant notablement inférieure au volume de la chambre de pression, et un élément d'entraînement (24) d'où le choc est susceptible d'être généré, le piston de travail (5) étant déplaçable vers l'intérieur dans la chambre de pression (13) par les chocs exercés successivement sur lui, chaque fois de la valeur d'une course de déplacement prédéterminée et étant en appui à l'encontre d'un déplacement en sens inverse, par l'intermédiaire d'un dispositif d'accouplement unidirectionnel linéaire, **caractérisé en ce que** le dispositif d'accouplement unidirectionnel linéaire est formé par au moins un élément d'étanchéité (18; 21; 65) élastique qui isole de façon étanche le piston de travail, ou bien un élément intermédiaire (58) éventuel servant à la transmission des chocs sur le piston de travail, en périphérie contre une paroi de délimitation afférente, et **en ce qu'**une résistance plus élevée est opposée au déplacement de rappel du piston de travail (5) que celle opposée au déplacement du piston de travail (5) vers l'intérieur de la chambre de pression (13).

2. Appareil éjecteur (1) selon la revendication 1, **caractérisé en ce que** sur le piston de travail (5) sont prévus deux éléments d'étanchéité (18, 21) élastiques disposés à distance l'un de l'autre, et dont le piston de travail (5) est isolé de façon étanche en périphérie par rapport à une paroi de délimitation afférente, et **en ce que** l'espacement entre les deux éléments d'étanchéité (18, 21) est supérieur à la course de travail totale du piston de travail (5), de sorte à former entre les deux éléments d'étanchéité (18, 21) une chambre annulaire (22) remplie de gaz, et entourant le piston de travail (5).

3. Appareil éjecteur (1) selon l'une des revendications 1 ou 2, **caractérisé en ce qu'**entre le piston de travail (5) et l'élément d'entraînement (24) est disposé un élément intermédiaire (58) transmettant le choc élastique de l'élément d'entraînement (24) au piston de travail (5), l'élément intermédiaire (58) étant séparé de celui-ci.

4. Appareil éjecteur (1) selon la revendication 3, **caractérisé en ce que** le piston de travail (5) et l'élément intermédiaire (58) sont fixés l'un à l'autre de façon désolidarisable par l'intermédiaire d'un élément d'accouplement (68).

5. Appareil éjecteur (1) selon la revendication 3 ou 4, **caractérisé en ce que** le dispositif d'accouplement unidirectionnel linéaire est prévu sur le piston de travail (5) et/ou sur l'élément intermédiaire (58).

6. Appareil éjecteur (1) selon l'une des revendications 3 à 5, **caractérisé en ce que** le dispositif d'accouplement unidirectionnel linéaire est formé par au moins deux éléments d'étanchéité (18, 21; 65) élastiques, dont l'un isole le piston de travail (5) et l'autre isole l'élément intermédiaire (58) en périphérie contre la paroi de délimitation chaque fois afférente.

7. Appareil éjecteur (1) selon l'une des revendications précédentes, **caractérisé en ce que** le dispositif d'accouplement unidirectionnel linéaire comporte comme élément d'étanchéité une bague torique (18, 21) susceptible d'être comprimée à l'aide d'une bague de serrage (16) réglable sous l'effet d'une pression radiale contre le piston de travail (5) ou contre l'élément intermédiaire (58), dans sa direction axiale.

8. Appareil éjecteur (1) selon la revendication 7, **caractérisé en ce que** le dispositif d'accouplement unidirectionnel linéaire comporte une autre bague torique (21) disposée à distance axiale du premier élément torique (18) à l'aide d'une douille d'espacement (19) disposée au voisinage de la bague de serrage (16) réglable, et qui est susceptible d'être comprimée dans sa direction axiale à l'aide de la bague de serrage (16) réglable par transmission de force de la douille d'espacement (18) avec pression radiale contre le piston de travail (5) ou contre l'élément intermédiaire (58).

9. Appareil éjecteur selon l'une des revendications 1 à 8 **caractérisé par** une partie de tête d'appareil éjecteur (2) dans lequel le piston de travail (5) et au moins partiellement la chambre de pression (13) sont prévus, et une partie d'entraînement d'appareil éjecteur (23) dans laquelle sont prévus l'élément d'entraînement (24) et /ou l'élément intermédiaire (58), sachant que la partie de tête d'appareil éjecteur(2) et la partie d'entraînement d'appareil éjecteur(23) soient réalisées sous la forme d'unités d'appareil éjecteurs autonomes fixées l'une à l'autre par un accouplement désolidarisable.

10. Appareil éjecteur (1) selon la revendication 9, **caractérisé en ce que** la chambre de pression (13) est formée au moins partiellement par un espace creux (4) réalisé dans la partie de tête d'appareil éjecteur (2), espace creux dans lequel est logé également le piston de travail (5), le fond de l'espace creux (4) étant de forme correspondante à l'extrémité frontale (51), tournée vers lui, du piston de travail (5).

11. Appareil éjecteur (1) selon la revendication 9 ou 10, **caractérisé en ce que** la partie de tête d'appareil éjecteur (2) est réalisée sous la forme d'article à jeter après usage et remplaçable.

12. Appareil éjecteur (1) selon la revendication 11 ou 12, **caractérisé en ce que** la partie tête d'appareil éjecteur (2) est réalisée sous la forme d'une partie susceptible d'être rechargée, qui peut être enlevée de la partie d'entraînement d'appareil éjecteur (23) pour procéder au rechargement.

13. Appareil éjecteur (1) selon l'une des revendications 1 à 12 **caractérisé en ce que** l'élément d'entraînement (24) est réalisé sous la forme de pistons d'entraînement (24) susceptibles d'être entraînés par voie pneumatique, disposés dans un tube d'entraînement (25) présentant sur son tronçon d'extrémité tourné vers le piston de travail (5) une fente longitudinale, par laquelle l'espace intérieur du tube d'entraînement (24) est relié à un espace de contournement (40), par l'intermédiaire duquel le piston d'entraînement (24) est susceptible d'être rappelé à sa position initiale par l'air évacué à travers la fente longitudinale et comprimée, jusqu'à ce que le piston d'entraînement (24) atteigne l'extrémité de la fente longitudinale tournée vers le piston de travail (5).

14. Appareil éjecteur (1) selon l'une des revendications 1 à 13, **caractérisé par** un cathéter d'endoscope muni d'un canal conduisant les liquides, et à partir duquel la chambre de pression (13) est partiellement formée.

15. Appareil éjecteur selon la revendication 3, **caractérisé en ce que** l'élément intermédiaire (58) qui transmet le choc de l'élément d'entraînement (24) au piston de travail (5) est susceptible d'être réglé axialement, après la transmission du choc, dans la direction du piston de travail (5).

16. Appareil éjecteur selon la revendication (15), **caractérisé en ce que** l'élément intermédiaire (58) est déplaçable par l'élément d'entraînement (24) dans la direction du piston de travail (5) jusqu'à la mise en butée sur un élément de butée (100) qui de son côté est susceptible axialement dans la direction du piston de travail (5).

17. Appareil éjecteur selon la revendication 16, **caractérisé en ce qu'**entre l'élément de butée (100) et l'élément intermédiaire (58) est disposé un tampon amortisseur (102) amortissant de façon élastique la mise en butée de l'élément intermédiaire.

18. Appareil éjecteur selon la revendication 16 ou 17, **caractérisé en ce que** l'élément de butée (100) est susceptible d'être réglé axialement au moyen d'un filetage(101) et est en appui sans jeu sur le filetage par l'intermédiaire d'un ressort (103).

19. Appareil éjecteur selon l'une des revendications 16 à 18, **caractérisé en ce que** l'élément de butée (100) et l'élément intermédiaire (58) sont réglables par l'intermédiaire d'un tube d'entraînement (25), dans lequel l'élément d'entraînement (24) est disposé et est guidé de façon réglable.

20. Appareil éjecteur selon l'une des revendications 16 à 19, **caractérisé en ce qu'**est prévu un entraînement pas à pas réglant l'élément de butée (100).
